# EUROPÄISCHE PATENTSCHRIFT

(11) **EP 1 794 154 B1**
(45) Veröffentlichungstag und Bekanntmachung des Hinweises auf die Patenterteilung: **27.02.2013**
(21) Anmeldenummer: 05789491.7
(22) Anmeldetag: 15.09.2005
(51) Int. Cl.: C07D 413/12, C07D 401/12, A61K 31/538, A61P 11/00

(54) **HETEROARYLVERBINDUNGEN ALS BETAMIMETKIA ZUR BEHANDLUNG VON ATEMWEGSERKRANKUNGEN**
HETEROARYL COMPOUNDS FOR USE AS BETAMIMETICS IN THE TREATMENT OF RESPIRATORY DISEASES
COMPOSES HETEROARYLE EN TANT QUE BETAMIMETIQUES POUR TRAITER DES MALADIES DES VOIES RESPIRATOIRES

(30) Priorität: 21.09.2004 DE 102004045648
(43) Veröffentlichungstag der Anmeldung: 13.06.2007
(73) Patentinhaber: Boehringer Ingelheim International GmbH, 55216 Ingelheim am Rhein (DE); Boehringer Ingelheim Pharma GmbH & Co. KG, 55216 Ingelheim (DE)
(72) Erfinder: KONETZKI, Ingo, 88447 Warthausen (DE); BOUYSSOU, Thierry, 88447 Warthausen (DE); HOENKE, Christoph, 55218 Ingelheim (DE); LUSTENBERGER, Philipp, 4056 Basel (CH); SCHNAPP, Andreas, 88400 Biberach (DE); CEREDA, Enzo, I-15067 Novi Ligure (IT)
(74) Vertreter: Simon, Elke Anna Maria
(86) Internationale Anmeldenummer: PCT/EP2005/054595
(87) Internationale Veröffentlichungsnummer: WO 2006/032627

(56) Entgegenhaltungen:
- DE-A1- 2 833 140
- DE-A1-102004 003 428
- US-A- 4 154 829

## Beschreibung

Betamimetika (ß-adrenerge Substanzen) sind aus dem Stand der Technik bekannt. Beispielsweise sei diesbezüglich auf die Offenbarung der US 4,460,581 und 4,154,829 verwiesen, die Betamimetika zur Therapie unterschiedlichster Erkrankungen vorschlägt.

Zur medikamentösen Therapie von Erkrankungen ist es häufig wünschenswert, Arzneimittel mit einer längeren Wirkungsdauer bereitzustellen. Hierdurch kann in der Regel gewährleistet werden, dass die zur Erzielung des therapeutischen Effekts erforderliche Konzentration des Wirkstoffs im Organismus über einen längeren Zeitraum gegeben ist, ohne eine allzu häufige, wiederholte Gabe des Arzneimittels durchführen zu müssen. Die Applikation eines Wirkstoffs in längeren zeitlichen Abständen trägt im Übrigen in hohem Maße zum Wohlbefinden des Patienten bei.

Besonders wünschenswert ist die Bereitstellung eines Arzneimittels, welches therapeutisch sinnvoll durch einmalige Applikation pro Tag (Einmalgabe) eingesetzt werden kann. Eine einmal pro Tag erfolgende Anwendung hat den Vorteil, dass der Patient sich relativ schnell an die regelmäßige Einnahme des Medikaments zu bestimmten Tageszeiten gewöhnen kann.

Es ist daher Aufgabe der vorliegenden Erfindung, Betamimetika bereitzustellen, die einerseits bei der Therapie von Atemwegserkrankungen einen therapeutischen Nutzen entfalten und darüber hinaus durch eine längere Wirkdauer gekennzeichnet sind und somit zur Herstellung von Arzneimitteln mit längerer Wirksamkeit Verwendung finden können. Es ist insbesondere Aufgabe der vorliegenden Erfindung, Betamimetika bereitzustellen, die aufgrund ihrer langen Wirksamkeit zur Herstellung eines zur Therapie von Atemwegserkrankungen einmal täglich applizierbaren Arzneimittels eingesetzt werden können. Neben den vorstehend genannten Aufgaben ist es ferner Ziel der vorliegenden Erfindung, solche Betamimetika bereitzustellen, die nicht nur außerordentlich potent sind, sondern ferner durch ein hohes Maß an Selektivität gegenüber dem β₂-Adrenozeptor gekennzeichnet sind.

### BESCHREIBUNG DER ERFINDUNG

Überraschenderweise wurde gefunden, dass die vorstehend genannten Aufgaben durch Verbindungen der Formel **1** gelöst werden. Die vorliegende Erfindung betrifft deshalb Verbindungen der Formel **1**, 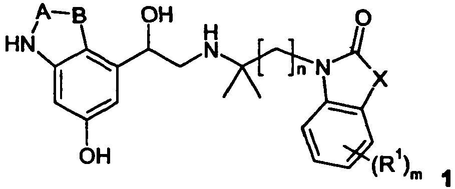 worin
- n: 1, 2, 3 oder 4;
- m: 1, 2, 3 oder 4;
- X: CH₂, CO, NR², S oder O;
- A: CO;
- B: eine zweibindige Gruppe ausgewählt aus der Gruppe bestehend aus O, S, CH₂, CR³R⁴-O, CR³R⁴-S, NR⁵, CR³R⁴-NR⁵, CH=CH oder CH₂-CH₂;
- R¹: H, C₁₋₆-Alkyl, C₂₋₆-Alkenyl, C₂₋₆-Alkinyl, C₃₋₆-Cycloalkyl, C₁₋₆-Haloalkyl, O- C₁₋₆-Haloalkyl, Halogen, OH, CN, NO₂, O-C₁₋₆-Alkyl, COOH oder COO-C₁₋₄-Alkyl;
- R²: H, C₁₋₆-Alkyl, C₁₋₄-Alkylen-C₆-C₁₀-aryl oder C₁₋₄-Alkylen-C₃₋₆-cycloalkyl, bevorzugt H oder C₁₋₆-Alkyl;
- R³: H oder C₁₋₆₋Alkyl;
- R⁴: H oder C₁₋₆₋Alkyl;
- R⁵: H oder C₁₋₆-Alkyl;
bedeuten, gegebenenfalls in Form der einzelnen Enantiomere, Mischungen der einzelnen Enantiomere oder Racemate, gegebenenfalls in Form ihrer Säureadditionssalze mit pharmakologisch unbedenklichen Säuren sowie gegebenenfalls in Form der Solvate und/oder Hydrate.

Bei mehrfach vorhandenen Resten R¹ (= m>1) können diese gleiche oder verschiedene Bedeutungen der für R¹ angegebenen Definitionen haben.

Bevorzugt sind die obigen Verbindungen der Formel 1, worin
- n: 1, 2 oder 3; bevorzugt 2 oder 3
- m: 1, 2, 3 oder 4; bevorzugt 1, 2 oder 3;
- X: CH₂, CO, NR², S oder O;
- A: CO;
- B: eine zweibindige Gruppe ausgewählt aus der Gruppe bestehend aus O, S, CH₂, CR³R⁴-O, CR³R⁴-S, NR⁵, CR³R⁴-NR⁵, CH=CH oder CH₂-CH₂;
- R¹: H, C₁₋₆-Alkyl, C₂₋₆-Alkenyl, C₂₋₆-Alkinyl, C₃₋₆-Cycloalkyl, C₁₋₆-Haloalkyl, O-C₁₋₆-Haloalkyl, Halogen, OH, CN, NO₂, O-C₁₋₆-Alkyl, COOH, COO-C₁₋₄-Alkyl; bevorzugt H, C₁₋₆-Alkyl, C₁₋₆-Haloalkyl, C₃₋₆-Cycloalkyl, Halogen, OH,CN, NO₂, O-C₁₋₆-Alkyl, COOH oder COO-C₁₋₄-Alkyl;
- R²: H, C₁₋₄-Alkyl, C₁₋₂-Alkylen-C₃₋₆-cycloalkyl, Phenylethyl oder Benzyl, bevorzugt H, C₁₋₄-Alkyl, C₃₋₆-Cycloalkyl-methyl, besonders bevorzugt H, Methyl oder Cyclopropylmethyl;
- R³: H oder C₁₋₄-Alkyl; bevorzugt H oder Methyl;
- R⁴: H oder C₁₋₄-Alkyl; bevorzugt H oder Methyl;
- R⁵: H oder C₁₋₄-Alkyl; bevorzugt H oder Methyl;
bedeuten, gegebenenfalls in Form der einzelnen Enantiomere, Mischungen der einzelnen Enantiomere oder Racemate, gegebenenfalls in Form ihrer Säureadditionssalze mit pharmakologisch unbedenklichen Säuren sowie gegebenenfalls in Form der Solvate und/oder Hydrate.

Bevorzugt sind die obigen Verbindungen der Formel **1** , worin
- n: 1, 2 oder 3; bevorzugt 2 oder 3
- m: 1, 2, 3 oder 4; bevorzugt 1,2 oder 3;
- X: CH₂, CO, NR², S oder O;
- A: CO;
- B: eine zweibindige Gruppe ausgewählt aus der Gruppe bestehend aus O, S, CH₂, CR³R⁴-O, CR³R⁴-S, NR⁵, CR³R⁴-NR⁵, CH=CH oder CH₂-CH₂;
- R¹: H, C₁₋₄-Alkyl, C₁₋₄-Haloalkyl, Cyclopropyl, Cyclohexyl, Halogen, OH, O-C₁₋₄-Alkyl, COOH oder COOMe;
- R²: H, C₁₋₄-Alkyl, C₃₋₆-Cycloalkyl-methyl, besonders bevorzugt H, Methyl oder Cyclopropylmethyl;
- R³: H oder C₁₋₄-Alkyl, bevorzugt H oder Methyl;
- R⁴: H oder C₁₋₄-Alkyl, bevorzugt H oder Methyl;
- R⁵: H oder C₁₋₄-Alkyl, bevorzugt H oder Methyl;
bedeuten, gegebenenfalls in Form der einzelnen Enantiomere, Mischungen der einzelnen Enantiomere oder Racemate, gegebenenfalls in Form ihrer Säureadditionssalze mit pharmakologisch unbedenklichen Säuren sowie gegebenenfalls in Form der Solvate und/oder Hydrate.

Bevorzugt sind die obigen Verbindungen der Formel **1**, worin
- n: 2 oder 3;
- m: 1, 2 oder 3;
- X: CH₂, CO, NR², S oder O;
- A: CO;
- B: eine zweibindige Gruppe ausgewählt aus der Gruppe bestehend aus O, S, CH₂, CR³R⁴-O, CR³R⁴-S, NR⁵, CR³R⁴-NR⁵, CH=CH oder CH₂-CH₂;
- R¹: H, Methyl, Ethyl, Propyl, CF₃, CH₂F, CH₂CF₃, Fluor, Chlor, Brom, OH, Methoxy, Ethoxy, COOH oder COOMe;
- R²: H, Methyl, Ethyl, Propyl, Cyclopropylmethyl, bevorzugt H oder Methyl;
- R³: H, Methyl, Ethyl oder Propyl, bevorzugt H;
- R⁴: H, Methyl, Ethyl oder Propyl, bevorzugt H;
- R⁵: H, Methyl, Ethyl oder Propyl, bevorzugt H;
bedeuten, gegebenenfalls in Form der einzelnen Enantiomere, Mischungen der einzelnen Enantiomere oder Racemate, gegebenenfalls in Form ihrer Säureadditionssalze mit pharmakologisch unbedenklichen Säuren sowie gegebenenfalls in Form der Solvate und/oder Hydrate.

Bevorzugt sind die obigen Verbindungen der Formel **1**, worin
- n: 2 oder 3;
- m: 1, 2 oder 3;
- X: CH₂, CO, NR², S oder O;
- A: CO;
- B: eine zweibindige Gruppe ausgewählt aus der Gruppe bestehend aus O, S, CH₂, CR³R⁴-O, CR³R⁴-S, NR⁵, CR³R⁴-NR⁵, CH=CH oder CH₂-CH₂;
- R¹: H, Methyl, Ethyl, Propyl, CF₃, CH₂F, CH₂CF₃, Fluor, Chlor, Brom, OH, Methoxy, Ethoxy, COOH oder COOMe;
- R²: H, Methyl, Ethyl oder Propyl;
- R³: H oder Methyl, bevorzugt H;
- R⁴: H oder Methyl, bevorzugt H;
- R⁵: H oder Methyl, bevorzugt H;
bedeuten, gegebenenfalls in Form der einzelnen Enantiomere, Mischungen der einzelnen Enantiomere oder Racemate, gegebenenfalls in Form ihrer Säureadditionssalze mit pharmakologisch unbedenklichen Säuren sowie gegebenenfalls in Form der Solvate und/oder Hydrate.

Bevorzugt sind die obigen Verbindungen der Formel **1**, worin
- n: 2 oder 3;
- m: 1, 2 oder 3;
- X: CH₂, CO, NR², S oder O;
- A: CO;
- B: eine zweibindige Gruppe ausgewählt aus der Gruppe bestehend aus CH₂-O, CH=CH oder CH₂-CH₂;
- R¹: H, Methyl, Ethyl, Propyl, CF₃, CH₂F, CH₂CF₃, Fluor, Chlor, Brom, OH, Methoxy, Ethoxy, COOH oder COOMe;
- R²: H, Methyl, Ethyl oder Propyl;
bedeuten, gegebenenfalls in Form der einzelnen Enantiomere, Mischungen der einzelnen Enantiomere oder Racemate, gegebenenfalls in Form ihrer Säureadditionssalze mit pharmakologisch unbedenklichen Säuren sowie gegebenenfalls in Form der Solvate und/oder Hydrate.

Bevorzugt sind die obigen Verbindungen der Formel **1**, worin
- n: 2 oder 3;
- m: 1 oder 2;
- X: CH₂, CO, NR², S oder O;
- A: CO;
- B: eine zweibindige Gruppe ausgewählt aus der Gruppe bestehend aus CH₂-O, CH=CH oder CH₂-CH₂;
- R¹: H, Methyl, Ethyl, Propyl, CF₃, CH₂F oder CH₂CF₃.
- R²: H, Methyl, Ethyl oder Propyl;
bedeuten, gegebenenfalls in Form der einzelnen Enantiomere, Mischungen der einzelnen Enantiomere oder Racemate, gegebenenfalls in Form ihrer Säureadditionssalze mit pharmakologisch unbedenklichen Säuren sowie gegebenenfalls in Form der Solvate und/oder Hydrate.

Bevorzugt sind die obigen Verbindungen der Formel **1**, worin
- n: 2 oder 3;
- m: 1;
- X: CH₂, CO, NR², S oder O;
- A: CO;
- B: eine zweibindige Gruppe ausgewählt aus der Gruppe bestehend aus CH₂-O, CH=CH oder CH₂-CH₂;
- R¹: H, Methyl oder CF_{3;}
- R²: H oder Methyl;
bedeuten, gegebenenfalls in Form der einzelnen Enantiomere, Mischungen der einzelnen Enantiomere oder Racemate, gegebenenfalls in Form ihrer Säureadditionssalze mit pharmakologisch unbedenklichen Säuren sowie gegebenenfalls in Form der Solvate und/oder Hydrate.

Bevorzugt sind die obigen Verbindungen der Formel **1**, worin
- X: NR² oder O; worin R² die oben genannte Bedeutung hat, bevorzugt H oder C₁₋₄-Alkyl; besonders bevorzugt H, Methyl, Ethyl oder Propyl, besonders bevorzugt H oder Methyl;
bedeutet, gegebenenfalls in Form der einzelnen Enantiomere, Mischungen der einzelnen Enantiomere oder Racemate, gegebenenfalls in Form ihrer Säureadditionssalze mit pharmakologisch unbedenklichen Säuren sowie gegebenenfalls in Form der Solvate und/oder Hydrate.

Bevorzugt sind die obigen Verbindungen der Formel **1**, worin
- n: 2 oder 3;
- m: 1;
- X: NR² oder O;
- A: CO;
- B: eine zweibindige Gruppe ausgewählt aus der Gruppe bestehend aus CH₂-O oder CH=CH;
- R¹: H, Methyl oder CF_{3;}
- R²: H oder Methyl;
bedeuten, gegebenenfalls in Form der einzelnen Enantiomere, Mischungen der einzelnen Enantiomere oder Racemate, gegebenenfalls in Form ihrer Säureadditionssalze mit pharmakologisch unbedenklichen Säuren sowie gegebenenfalls in Form der Solvate und/oder Hydrate.

Besonders bevorzugt sind die obigen Verbindungen der Formel **1**, worin
- n: 2;
- m: 1;
- X: NH;
- A: CO;
- B: eine zweibindige Gruppe CH₂-O;
- R¹: H, Methyl oder CF_{3;}
bedeutet, gegebenenfalls in Form der einzelnen Enantiomere, Mischungen der einzelnen Enantiomere oder Racemate, gegebenenfalls in Form ihrer Säureadditionssalze mit pharmakologisch unbedenklichen Säuren sowie gegebenenfalls in Form der Solvate und/oder Hydrate.

Besonders bevorzugt sind ferner die obigen Verbindungen der Formel **1**, worin
- X: NR²;

- R²: Cyclopropylmethyl, Cyclopropylethyl, Cyclopentylmethyl, Cyclopentylethyl, Cyclohexylmethyl oder Cycloheylethyl, bevprzugt Cyclopropylmethyl, Cyclopentylmethyl oder Cyclohexylmethyl, besonders bevorzugt Cyclopropylmethyl;
und worin die Reste n, m, A, B und R¹ die vorstehend genannten Bedeutungen haben können, gegebenenfalls in Form der einzelnen Enantiomere, Mischungen der einzelnen Enantiomere oder Racemate, gegebenenfalls in Form ihrer Säureadditionssalze mit pharmakologisch unbedenklichen Säuren sowie gegebenenfalls in Form der Solvate und/oder Hydrate.

Bevorzugt sind ferner Verbindungen der Formel 1, worin X CH₂ bedeutet und worin die Reste n, m, A, B und R¹ die vorstehend genannten Bedeutungen haben können, gegebenenfalls in Form der einzelnen Enantiomere, Mischungen der einzelnen Enantiomere oder Racemate, gegebenenfalls in Form ihrer Säureadditionssalze mit pharmakologisch unbedenklichen Säuren sowie gegebenenfalls in Form der Solvate und/oder Hydrate.

Bevorzugt sind ferner Verbindungen der Formel 1, worin X CO bedeutet und worin die Reste n, m, A, B und R¹ die vorstehend genannten Bedeutungen haben können, gegebenenfalls in Form der einzelnen Enantiomere, Mischungen der einzelnen Enantiomere oder Racemate, gegebenenfalls in Form ihrer Säureadditionssalze mit pharmakologisch unbedenklichen Säuren sowie gegebenenfalls in Form der Solvate und/oder Hydrate.

Bevorzugt sind ferner Verbindungen der Formel 1, worin X O bedeutet und worin die Reste n, m, A, B und R¹ die vorstehend genannten Bedeutungen haben können, gegebenenfalls in Form der einzelnen Enantiomere, Mischungen der einzelnen Enantiomere oder Racemate, gegebenenfalls in Form ihrer Säureadditionssalze mit pharmakologisch unbedenklichen Säuren sowie gegebenenfalls in Form der Solvate und/oder Hydrate.

Bevorzugt sind ferner Verbindungen der Formel 1, worin X S bedeutet und worin die Reste n, m, A, B und R¹ die vorstehend genannten Bedeutungen haben können, gegebenenfalls in Form der einzelnen Enantiomere, Mischungen der einzelnen Enantiomere oder Racemate, gegebenenfalls in Form ihrer Säureadditionssalze mit pharmakologisch unbedenklichen Säuren sowie gegebenenfalls in Form der Solvate und/oder Hydrate.

Bevorzugt sind ferner Verbindungen der Formel 1, worin X NR₂ bedeutet und worin die Reste n, m, A, B, R¹ und R² die vorstehend genannten Bedeutungen haben können, gegebenenfalls in Form der einzelnen Enantiomere, Mischungen der einzelnen Enantiomere oder Racemate, gegebenenfalls in Form ihrer Säureadditionssalze mit pharmakologisch unbedenklichen Säuren sowie gegebenenfalls in Form der Solvate und/oder Hydrate.

Bevorzugt sind ferner Verbindungen der Formel 1, worin X NH bedeutet und worin die Reste n, m, A, B und R¹ die vorstehend genannten Bedeutungen haben können, gegebenenfalls in Form der einzelnen Enantiomere, Mischungen der einzelnen Enantiomere oder Racemate, gegebenenfalls in Form ihrer Säureadditionssalze mit pharmakologisch unbedenklichen Säuren sowie gegebenenfalls in Form der Solvate und/oder Hydrate.

Verbindungen der Formel **1**, worin A CO und B CH₂-O bedeuten sind gekennzeichnet durch die allgemeine Formel **1.1**.

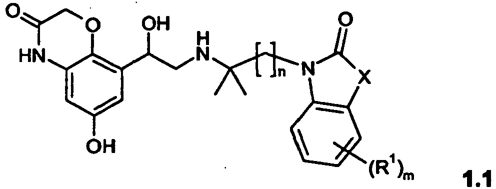

Ein bevorzugter Aspekt der vorliegenden Erfindung betrifft Verbindungen der Formel **1.1** worin n, m, X und R¹ die vorstehend genannten Bedeutungen haben können, gegebenenfalls in Form der einzelnen Enantiomere, Mischungen der einzelnen Enantiomere oder Racemate, gegebenenfalls in Form ihrer Säureadditionssalze mit pharmakologisch unbedenklichen Säuren sowie gegebenenfalls in Form der Solvate und/oder Hydrate.

Verbindungen der Formel **1**, worin A CO und B CH=CH bedeuten sind gekennzeichnet durch die allgemeine Formel **1.2**.

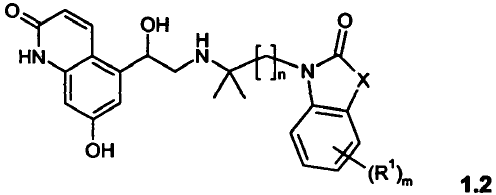

Ein bevorzugter Aspekt der vorliegenden Erfindung betrifft Verbindungen der Formel **1.2** worin n, m, X und R¹ die vorstehend genannten Bedeutungen haben können, gegebenenfalls in Form der einzelnen Enantiomere, Mischungen der einzelnen Enantiomere oder Racemate, gegebenenfalls in Form ihrer Säureadditionssalze mit pharmakologisch unbedenklichen Säuren sowie gegebenenfalls in Form der Solvate und/oder Hydrate.

Verbindungen der Formel **1**, worin A CO und B CH₂-CH₂ bedeuten, sind gekennzeichnet durch die allgemeine Formel **1.3**.

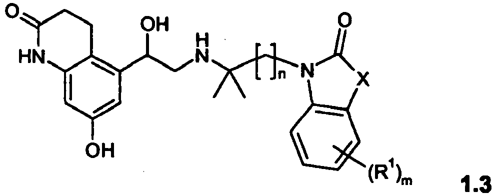

Ein bevorzugter Aspekt der vorliegenden Erfindung betrifft Verbindungen der Formel **1.3** worin n, m, X und R¹ die vorstehend genannten Bedeutungen haben können, gegebenenfalls in Form der einzelnen Enantiomere, Mischungen der einzelnen Enantiomere oder Racemate, gegebenenfalls in Form ihrer Säureadditionssalze mit pharmakologisch unbedenklichen Säuren sowie gegebenenfalls in Form der Solvate und/oder Hydrate.

Verbindungen der Formel **1**, worin A CO und B O bedeuten, sind gekennzeichnet durch die allgemeine Formel **1.4**.

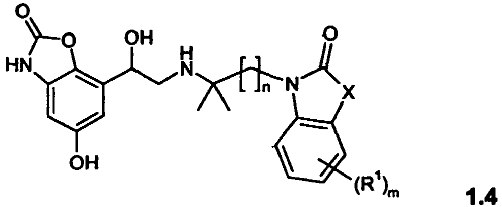

Ein bevorzugter Aspekt der vorliegenden Erfindung betrifft Verbindungen der Formel **1.4** worin n, m, X und R¹ die vorstehend genannten Bedeutungen haben können, gegebenenfalls in Form der einzelnen Enantiomere, Mischungen der einzelnen Enantiomere oder Racemate, gegebenenfalls in Form ihrer Säureadditionssalze mit pharmakologisch unbedenklichen Säuren sowie gegebenenfalls in Form der Solvate und/oder Hydrate.

Verbindungen der Formel **1**, worin A CO, B CR³R⁴-O und R³ bzw. R⁴ Methyl bedeuten, sind gekennzeichnet durch die allgemeine Formel **1.5**.

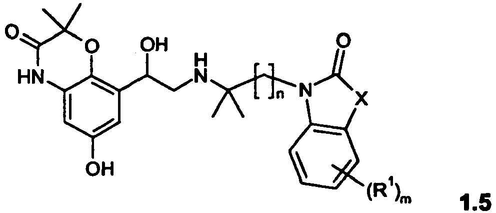

Ein bevorzugter Aspekt der vorliegenden Erfindung betrifft Verbindungen der Formel **1.5** worin n, m, X und R¹ die vorstehend genannten Bedeutungen haben können, gegebenenfalls in Form der einzelnen Enantiomere, Mischungen der einzelnen Enantiomere oder Racemate, gegebenenfalls in Form ihrer Säureadditionssalze mit pharmakologisch unbedenklichen Säuren sowie gegebenenfalls in Form der Solvate und/oder Hydrate.

Besonders bevorzugt sind ferner Verbindungen der Formel **1**, die ausgewählt sind aus der Gruppe bestehend aus 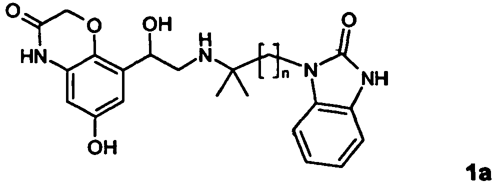 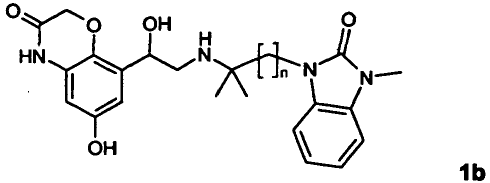 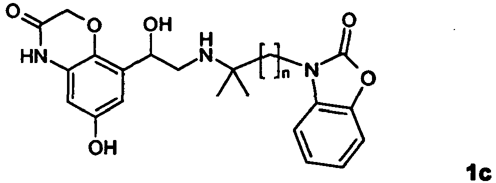 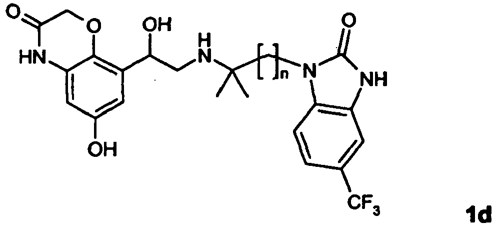 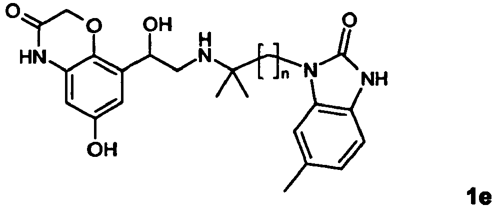 worin für **1a** n = 2 oder 3 ist kann und für **1b, 1c, 1d** und **1e** n = 2 ist und die Verbindungen gegebenenfalls in Form der einzelnen Enantiomere, Mischungen der einzelnen Enantiomere oder Racemate, gegebenenfalls in Form ihrer Säureadditionssalze mit pharmakologisch unbedenklichen Säuren sowie gegebenenfalls in Form der Solvate und/oder Hydrate vorliegen.

Ein weiterer Aspekt der vorliegenden Erfindung betrifft die vorstehend genannten neuen Verbindungen der Formel **1** in Form der einzelnen optischen Isomeren, Mischungen der einzelnen Enantiomeren oder Racemate. Besonders bevorzugt sind dabei Verbindungen der Formel **1** in Form der enantiomerenreinen Verbindungen, wobei die R-Enantiomere der Verbindungen der Formel **1** erfindungsgemäß von herausragender Bedeutung sind. Die R-Enantiomere der Verbindungen der Formel **1** sind darstellbar durch die allgemeine Formel **R-1** 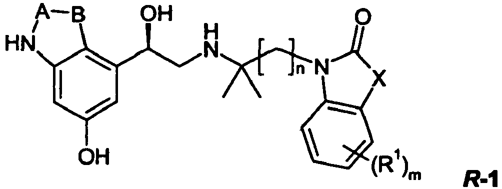 worin die Reste n, m, A, B, X und R¹ die vorstehend genannten Bedeutungen haben können. Besonders bevorzugt sind hiervon ferner Verbindungen der Formel ***R*-1**, die ausgewählt sind aus der Gruppe bestehend aus 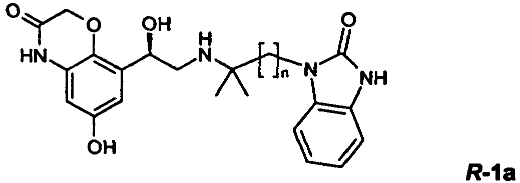 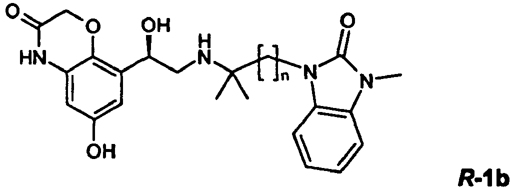 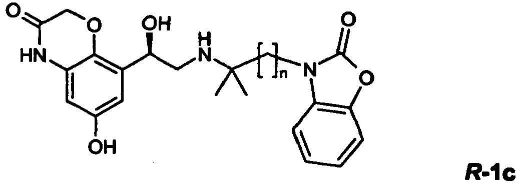 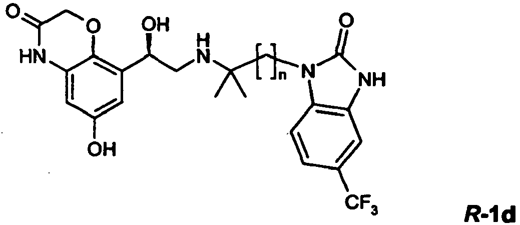 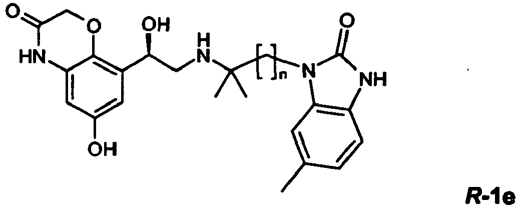 worin bei ***R*-1a** und ***R*-1c** n = 2 oder 3 und bei ***R*-1b, *R*-1d** und ***R*-1e** n = 2 bedeutet und die Verbindungen gegebenenfalls in Form der einzelnen Enantiomere, Mischungen der einzelnen Enantiomere oder Racemate, gegebenenfalls in Form ihrer Säureadditionssalze mit pharmakologisch unbedenklichen Säuren sowie gegebenenfalls in Form der Solvate und/oder Hydrate vorliegen.

Verfahren zur Auftrennung von Racematen in die jeweiligen Enantiomere sind im Stand der Technik bekannt und können zur Darstellung der enantiomerenreinen R- bzw. S-Enantiomere der Verbindungen der Formel **1** in analoger Art und Weise zur Anwendung gelangen.

Ein weiterer Aspekt der vorliegenden Erfindung betrifft die vorstehend genannten neuen Verbindungen der Formel **1** als Arzneimittel. Die vorliegende Erfindung betrifft ferner die Verwendung der vorstehend genannten Verbindungen der Formel **1** zur Herstellung eines Arzneimittels zur Behandlung von Atemwegserkrankungen.

Die vorliegende Erfindung betrifft bevorzugt die Verwendung der vorstehend genannten Verbindungen der Formel **1** zur Herstellung eines Arzneimittels zur Behandlung von Atemwegserkrankungen, die ausgewählt sind aus der Gruppe bestehend aus Obstruktive Lungenerkrankungen unterschiedlicher Genese, Lungenemphyseme unterschiedlicher Genese, Restriktive Lungenerkrankungen, Interstitielle Lungenerkrankungen, Zystische Fibrose, Bronchitiden unterschiedlicher Genese, Bronchiektasen, ARDS (adult respiratory distress syndrom) und alle Formen des Lungenödems.

Bevorzugt ist die Verwendung von Verbindungen der Formel **1** zur Herstellung eines Arzneimittels zur Behandlung von Obstruktiven Lungenerkrankungen, die ausgewählt sind aus der Gruppe bestehend aus COPD (chronisch obstruktive pulmonale Erkrankung), Asthma Bronchiale, pädiatrisches Asthma, schweres Asthma, akuter Asthma-Anfall und chronische Bronchitis, wobei die Verwendung zur Herstellung eines Arzneimittels zur Behandlung von Asthma Bronchiale erfindungsgemäß besonders bevorzugt ist.

Bevorzugt ist ferner die Verwendung von Verbindungen der Formel **1** zur Herstellung eines Arzneimittels zur Behandlung von Lungenemphysemen die ihren Ursprung haben in COPD (chronisch obstruktive pulmonale Erkrankung) oder α1-Proteinase-Inhibitor-Mangel.

Bevorzugt ist ferner die Verwendung von Verbindungen der Formel **1** zur Herstellung eines Arzneimittels zur Behandlung von Restriktiven Lungenerkrankungen, die ausgewählt sind aus der Gruppe bestehend aus Allergische Alveolitis, durch berufliche Noxen ausgelöste restriktive Lungenerkrankungen wie Asbestose oder Silikose und Restriktion aufgrund von Lungentumoren, wie beispielsweise Lymphangiosis carcinomatosa, bronchoalveoläres Karzinom und Lymphome.

Bevorzugt ist ferner die Verwendung von Verbindungen der Formel **1** zur Herstellung eines Arzneimittels zur Behandlung von Interstitiellen Lungenerkrankungen, die ausgewählt sind aus der Gruppe bestehend aus infektiös bedingte Pneumonien, wie beispielsweise aufgrund einer Infektion mit Viren, Bakterien, Pilzen, Protozoen, Helminthen oder anderen Erregern, Pneumonitis unterschiedlicher Genese, wie beispielsweise Aspiration und Linksherzinsuffizienz, Strahlen-induzierte Pneumonitis oder Fibrose, Kollagenosen, wie beispielsweise Lupus erythematodes, systemische Sklerodermie oder Sarkoidose, Granulomatosen, wie beispielsweise Morbus Boeck, idiopathische interstitielle Pneumonie oder idiopathische pulmonäre Fibrose (IPF).

Bevorzugt ist ferner die Verwendung von Verbindungen der Formel **1** zur Herstellung eines Arzneimittels zur Behandlung von Zystischer Fibrose bzw. Mukoviszidose.

Bevorzugt ist ferner die Verwendung von Verbindungen der Formel **1** zur Herstellung eines Arzneimittels zur Behandlung von Bronchitiden, wie beispielsweise Bronchitis aufgrund bakterieller oder viraler Infektion, Allergische Bronchitis und Toxische Bronchitis.

Bevorzugt ist ferner die Verwendung von Verbindungen der Formel **1** zur Herstellung eines Arzneimittels zur Behandlung von Bronchiektasen.

Bevorzugt ist ferner die Verwendung von Verbindungen der Formel **1** zur Herstellung eines Arzneimittels zur Behandlung von ARDS (adult respiratory distress syndrom).

Bevorzugt ist ferner die Verwendung von Verbindungen der Formel **1** zur Herstellung eines Arzneimittels zur Behandlung von Lungenödemen, beispielsweise toxischer Lungenödeme nach Aspiration oder Inhalation von toxischen Substanzen und Fremdstoffen.

Besonders bevorzugt betrifft die vorliegende Erfindung die Verwendung der Verbindungen der Formel **1** zur Herstellung eines Arzneimittels zur Behandlung von Asthma oder COPD. Von besonderer Bedeutung ist ferner die vorstehend genannte Verwendung von Verbindungen der Formel **1** zur Herstellung eines Arzneimittels zur einmal täglichen Behandlung von entzündlichen und obstruktiven Atemwegserkrankungen, besonders bevorzugt zur einmal täglichen Behandlung von Asthma oder COPD.

Ferner betrifft die vorliegende Erfindung ein Verfahren zur Behandlung der vorstehend genannten Erkrankungen, dadurch gekennzeichnet, daß eine oder mehrere der vorstehend genannten Verbindungen der Formel **1** in therapeutisch wirksamen Mengen appliziert werden. Die vorliegende Erfindung betrifft bevorzugt Verfahren zur Behandlung von Asthma oder COPD, dadurch gekennzeichnet, daß eine oder mehrere der vorstehend genannten Verbindungen der Formel **1** in therapeutisch wirksamen Mengen einmal täglich appliziert werden.

Ein weiterer Aspekt der vorliegenden Erfindung betrifft die vorstehend genannten Verbindungen der Formel **1** in Form ihrer Säureadditionssalze mit pharmakologisch unbedenklichen Säuren sowie gegebenenfalls in Form der Solvate und/oder Hydrate.

Unter Säureadditionssalzen mit pharmakologisch unbedenklichen Säuren werden dabei beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden.

Unter dem Begriff "C₁₋₆-Alkyl" (auch soweit sie Bestandteil anderer Reste sind) werden verzweigte und unverzweigte Alkylgruppen mit 1 bis 6 Kohlenstoffatomen verstanden und unter dem Begriff "C₁₋₄-Alkyl" verzweigte und unverzweigte Alkylgruppen mit 1 bis 4 Kohlenstoffatomen verstanden. Bevorzugt sind Alkylgruppen mit 1 bis 4 Kohlenstoffatomen. Beispielsweise werden hierfür genannt: Methyl, Ethyl, *n*-Propyl, *iso*-Propyl, *n-*Butyl, *iso*-Butyl, *sec*-Butyl, *tert*-Butyl, *n*-Pentyl, *iso*-Pentyl, *neo*-Pentyl oder Hexyl. Gegebenenfalls werden für vorstehend genannten Gruppen auch die Abkürzungen Me, Et, *n*-Pr, *i*-Pr, *n*-Bu, *i*-Bu, *t*-Bu, etc. verwendet. Sofern nicht anders beschrieben, umfassen die Definitionen Propyl, Butyl, Pentyl und Hexyl alle denkbaren isomeren Formen der jeweiligen Reste. So umfasst beispielsweise Propyl *n*-Propyl und *iso*-Propyl, Butyl umfasst *iso*-Butyl, *sec*-Butyl und *tert*-Butyl etc.
Alkylengruppen sind im Rahmen der vorliegenden Erfindung verbrückende Alkylgruppen, das heißt Alkylgruppen, die mit zwei weiteren Gruppen verknüpft sind.

Unter dem Begriff "C₂₋₆-Alkenyl" (auch soweit sie Bestandteil anderer Reste sind) werden verzweigte und unverzweigte Alkenylgruppen mit 2 bis 6 Kohlenstoffatomen und unter dem Begriff "C₂₋₄-Alkenyl" verzweigte und unverzweigte Alkenylgruppen mit 2 bis 4 Kohlenstoffatomen verstanden, soweit sie mindestens eine Doppelbindung aufweisen. Bevorzugt sind Alkenylgruppen mit 2 bis 4 Kohlenstoffatome. Beispielsweise werden hierfür genannt: Ethenyl oder Vinyl, Propenyl, Butenyl, Pentenyl, oder Hexenyl. Sofern nicht anders beschrieben, umfassen die Definitionen Propenyl, Butenyl, Pentenyl und Hexenyl alle denkbaren isomeren Formen der jeweiligen Reste. So umfasst beispielsweise Propenyl 1-Propenyl und 2-Propenyl, Butenyl umfasst 1-, 2- und 3-Butenyl, 1-Methyl-1-propenyl, 1-Methyl-2-propenyl etc.

Unter dem Begriff "C₂₋₆-Alkinyl" (auch soweit sie Bestandteil anderer Reste sind) werden verzweigte und unverzweigte Alkinylgruppen mit 2 bis 6 Kohlenstoffatomen und unter dem Begriff "C₂₋₄-Alkinyl" verzweigte und unverzweigte Alkinylgruppen mit 2 bis 4 Kohlenstoffatomen verstanden, soweit sie mindestens eine Dreifachbindung aufweisen. Bevorzugt sind Alkinylgruppen mit 2 bis 4 Kohlenstoffatome. Beispielsweise werden hierfür genannt: Ethinyl, Propinyl, Butinyl, Pentinyl, oder Hexinyl. Sofern nicht anders beschrieben, umfassen die Definitionen Propinyl, Butinyl, Pentinyl und Hexinyl alle denkbaren isomeren Formen der jeweiligen Reste. So umfasst beispielsweise Propinyl 1-Propinyl und 2-Propinyl, Butinyl umfasst 1-, 2- und 3-Butinyl, 1-Methyl-1-propinyl, 1-Methyl-2-propinyl etc.

Unter dem Begriff "C₃₋₆-Cycloalkyl" (auch soweit sie Bestandteil anderer Reste sind) werden cyclische Alkylgruppen mit 3 bis 6 Kohlenstoffatomen verstanden. Beispielsweise werden hierfür genannt: Cyclopropyl, Cyclopentyl oder Cyclohexyl. Soweit nicht anders beschrieben, können die cyclischen Alkylgruppen substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod. Alkylencycloalkyl-Gruppen sind Cycloalkyle, die über eine Alkylenbrücke verknüpft sind. Hierzu zählt insbesondere Cyclopropylmethyl.

Unter dem Begriff "C₁₋₆-Haloalkyl" (auch soweit sie Bestandteil anderer Reste sind) werden verzweigte und unverzweigte Alkylgruppen mit 1 bis 6 Kohlenstoffatomen verstanden, die mit einem oder mehreren Halogenatomen substituiert sind. Unter dem Begriff "C₁₋₄-Alkyl" verzweigte und unverzweigte Alkylgruppen mit 1 bis 4 Kohlenstoffatomen verstanden, die mit einem oder mehreren Halogenatomen substituiert sind. Bevorzugt sind Alkylgruppen mit 1 bis 4 Kohlenstoffatomen. Bevorzugte Halogenatome sind Fluor, Chlor, besonders bevorzugt Fluor. Beispielsweise werden hierfür genannt: CF₃, CHF₂, CH₂F, CH₂CF₃.

Unter dem Begriff "C₆₋₁₀-Aryl" (auch soweit sie Bestandteil anderer Reste sind) werden aromatische Cyclen verstanden, die 6 bis 10 Kohelenstoffzentren enthalten können. Beispielsweise seien genannt Phenyl oder Naphthyl. Alkylenaryl-Gruppen sind ArylGruppen, die über eine Alkylenbrücke verknüpft sind. Hierzu zählt insbesondere Benzyl.

Halogen steht im Rahmen der vorliegenden Erfindung für Fluor, Chlor, Brom oder Jod. Sofern nicht gegenteilig angegeben, gelten Fluor, Chlor und Brom als bevorzugte Halogene.

Verbindungen der allgemeinen Formel **1** können Säuregruppen besitzen, hauptsächlich Carboxylgruppen, und/oder basische Gruppen wie z.B. Aminofunktionen. Verbindungen der allgemeinen Formel **1** können deshalb als innere Salze, als Salze mit pharmazeutisch verwendbaren anorganischen Säuren wie Salzsäure, Schwefelsäure, Phosphorsäure, Sulfonsäure oder organischen Säuren (wie beispielsweise Maleinsäure, Fumarsäure, Zitronensäure, Weinsäure oder Essigsäure) oder als Salze mit pharmazeutisch verwendbaren Basen wie Alkali- oder Erdalkalimetallhydroxiden oder Carbonaten, Zink- oder Ammoniumhydroxiden oder organischen Aminen wie z.B. Diethylamin, Triethylamin, Triethanolamin u.a. vorliegen.

Wie vorstehend genannt, können die Verbindungen der Formel **1** in ihre Salze, insbesondere für die pharmazeutische Anwendung, in ihre physiologisch und pharmakologisch verträglichen Salze überführt werden. Diese Salze können einerseits als physiologisch und pharmakologisch verträgliche Säureadditionssalze der Verbindungen der Formel **1** mit anorganischen oder organischen Säuren vorliegen. Zur Darstellung der Säureadditionssalze kommen beispielsweise Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure oder Maleinsäure in Betracht. Ferner können Mischungen der vorgenannten Säuren eingesetzt werden. Zur Darstellung der Alkali- und Erdalkalimetallsalze der Verbindung der Formel **1** kommen vorzugsweise die Alkali- und Erdalkalihydroxide und -hydride in Betracht, wobei die Hydroxide und Hydride der Alkalimetalle, besonders des Natriums und Kaliums bevorzugt, Natrium- und Kaliumhydroxid besonders bevorzugt sind.

Gegebenenfalls können die Verbindungen der allgemeinen Formel (1) in ihre Salze, insbesondere für die pharmazeutische Anwendung, in ihre pharmakologisch unbedenklichen Säureadditionssalze mit einer anorganischen oder organischen Säure, überführt werden. Als Säuren kommen hierfür beispielsweise Bernsteinsäure, Bromwasserstoffsäure, Essigsäure, Fumarsäure, Maleinsäure, Methansulfonsäure, Milchsäure, Phosphorsäure, Salzsäure, Schwefelsäure, Weinsäure oder Zitronensäure in Betracht. Ferner können Mischungen der vorgenannten Säuren eingesetzt werden.

Gegenstand der Erfindung sind die jeweiligen Verbindungen gegebenenfalls in Form der einzelnen optischen Isomeren, Mischungen der einzelnen Enantiomeren oder Racemate, in Form der Tautomere sowie in Form der freien Basen oder der entsprechenden Säureadditionssalze mit pharmakologisch unbedenklichen Säuren - wie beispielsweise Säureadditionssalze mit Halogenwasserstoffsäuren - beispielsweise Chlor- oder Bromwasserstoffsäure - oder organische Säuren - wie beispielsweise Oxal-, Fumar-, Diglycol- oder Methansulfonsäure.

Die erfindungsgemäßen Verbindungen können als Racemate vorliegen, sie können aber auch als reine Enantiomere, d.h. in (R)- oder (S)-Form gewonnen werden. Bevorzugt sind Verbindungen die als Racemate bzw. als (R)-Form vorliegen.

Die Herstellung der erfindungsgemäßen Verbindungen kann in Analogie zu im Stand der Technik bereits bekannten Vorgehensweisen erfolgen. Geeignete Herstellverfahren sind beispielsweise aus der US 4460581 und 4,154,829 bekannt, auf die an dieser Stelle vollinhaltlich Bezug genommen wird.

Die nachstehenden Beispiele dienen der weitergehenden Veranschaulichung und Verdeutlichung der vorliegenden Erfindung ohne deren Gegenstand allerdings auf die exemplarisch erläuterten Beispiele zu beschränken.

### BEISPIELE

### SYNTHESE VON ZWISCHENSTUFEN

### Zwischenstufe 1: (3-Amino-3-methyl-butyl)-carbaminsäure-tert-butylester

23.6 g (117 mmol) (1,1-Dimethyl-3-oxo-propyl)-carbaminsäure-tert-butylester in 700 mL ethanolischer Ammoniak-Lösung werden in Gegenwart von 3.5 g Raney-Nickel bei Rautemperatur mit 3 bar Wasserstoffdruck behandelt bis kein Edukt mehr dünnschichtchromatographisch nachgewiesen werden kann. Der Katalysator wird abfiltriert und das Lösungsmittel destillativ entfernt. Dunkelgrünes Öl. Ausbeute: 22.7 g (96%); Massenspektroskopie: [M+H]⁺ = 203.

### Zwischenstufe 2: 1-(3-Amino-1,1-dimethyl-proypl)-6-methyl-1,3-dihydro-benzoimidazol-2-on

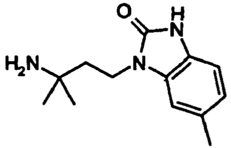

### a) [3-Methyl-3-(5-methyl-2-nitro-phenylamino)-butyl]-carbaminsäure-tert-butylester

2.0 g (12.9 mmol) 3-Fluor-4-nitro-toluol, 2.6 g (13.0 mmol) (3-Amino-3-methyl-butyl)-carbaminsäure-tert-butylester und 2.3 g (16.8 mmol) Kaliumcarbonat werden über Nacht bei Raumtemperatur in 20 mL DMF gerührt. Das Lösungsmittel wird abdestilliert und der Rückstand mit Ethylacetat versetzt. Man wäscht wiederholt mit Wasser, trocknet mit Natriumsulfat und entfernt das Lösungsmittel. 4.8 g gelbes Öl. Massenspektroskopie: [M+H]⁺ = 338.

### b) [3-(2-Amino-5-methyl-phenylamino)-3-methyl-butyl]-carbaminsäure-tert-butylester

4.71 g (14.0 mmol) [3-Methyl-3-(5-methyl-2-nitro-phenylamino)-butyl]-carbaminsäure-tert-butylester werden in 110 mL Methanol gelöst und in Gegenwart von 340 mg Palladium auf Kohle (10%ig) bei Raumtemperatur hydriert. Anschließend wird der Katalysator abgetrennt und das Lösungsmittel abdestilliert. Brauner Feststoff. Ausbeute: 3.72 g (87%); Massenspektroskopie: [M+H]⁺ = 308.

### c) [3-Methyl-3-(6-methyl-2-oxo-2,3-dihydro-benzoimidazol-1-yl)-butyl]-carbaminsäure-tert-butylester

1.76 g (5.7 mmol) [3-(2-Amino-5-methyl-phenylamino)-3-methyl-butyl]-carbaminsäure-tert-butylester werden in 35 mL THF gelöst, mit 2.1 g (12.7 mmol) 1,1'-Carbonyldi-(1,2,4-triazol) versetzt und über Nacht gerührt. Das Lösungsmittel wird abdestilliert und der Rückstand in Ethylacetat gelöst. Die Lösung wird nacheinander mit KaliumhydrogensulfatLösung und Natriumchlorid-Lösung gewaschen und mit Natriumsulfat getrocknet. Der Rückstand wird chromatographiert (Kieselgel; Dichlormethan mit 0-16% Methanol:Ammoniak = 9:1) und das so gewonnene Rohprodukt mit Diethylether verrührt. Leicht gelber Feststoff. Ausbeute: 1.12 g (59%); Massenspektroskopie: [M+H]⁺ = 334.

### d) 1-(3-Amino-1,1-dimethyl-proypl)-6-methyl-1,3-dihydro-benzoimidazol-2-on

Eine Lösung von 1.50 g (4.5 mmol) [3-Methyl-3-(6-methyl-2-oxo-2,3-dihydrobenzoimidazol-1-yl)-butyl]-carbaminsäure-tert-butylester in 100 mL Dioxan wird mit 10 mL 4 molarer Salzsäure in Dioxan versetzt und dann für 90 Minuten auf 90°C erwärmt, wobei ein weißer Niederschlag ausfällt. Nach Abkühlung auf Raumtemperatur wird das Lösungsmittel abdestilliert und der Rückstand in Diethylether verrührt. Weißer Feststoff. Ausbeute: 1.04 g (86%; Hydrochlorid) Massenspektroskopie: [M+H]⁺ = 234.

### Zwischenstufe 3:1-(3-Amino-3-methyl-butyl)-5-trifluormethyl-1,3-dihydro-benzoimidazol-2-on

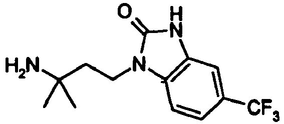

### a) [3-Methyl-3-(2-nitro-4-trifluormethyl-phenylamino)-butyl]-carbaminsäure-tert-butylester

Die Herstellung erfolgt in Analogie zur Arbeitsvorschrift 2a) aus insgesamt 3.25 g (15.5 mmol) 1-Fluor-2-nitro-4-trifluormethyl-benzol und 2.74 g (13.5 mmol) (3-Amino-3-methylbutyl)-carbaminsäure-tert-butylester. 6.1 g gelbes Öl. Massenspektroskopie: [M+H]⁺ = 392.

### b) [3-(2-Amino-4-trifluormethyl-phenylamino)-1,1-dimethyl-propyl]-carbaminsäure-tert-butylester

6.10 g (15.6 mmol) [3-Methyl-3-(2-nitro-4-trifluormethyl-phenylamino)-butyl]-carbaminsäure-tert-butylester werden in Analogie zur Arbeitsvorschift 2b) hydriert. Ausbeute: 5.05 g (90%); Massenspektroskopie: [M+H]⁺ = 362.

### c) [1,1-Dimethyl-3-(2-oxo-5-trifluormethyl-2,3-dihydro-benzoimidazol-1-yl)-propyl]-carbaminsäure-tert-butylester

5.00 g (13.8 mmol) [3-(2-Amino-4-trifluormethyl-phenylamino)-1,1-dimethyl-propyl]-carbaminsäure-tert-butylester und 6.73 g (41.5 mmol) 1,1'-Carbonyldiimidazol werden analog zur Arbeitsvorschrift 2c) umgesetzt und aufgearbeitet. Weißer Feststoff. Ausbeute: 4.18 g (78%); Massenspektroskopie: [M-H]⁺ = 386.

### d) 1-(3-Amino-3-methyl-butyl)-5-trifluormethyl-1,3-dihydro-benzoimidazol-2-on

Herstellung in Analogie zur Arbeitsvorschrift 2d) aus 2.89 g (7.5 mmol) [1,1-Dimethyl-3-(2-oxo-5-trifluormethyl-2,3-dihydro-benzoimidazol-1-yl)-propyl]-carbaminsäure-tert-butylester. Ausbeute: 1.60 g (66%); Massenspektroskopie: [M+H]⁺ = 288.

### Zwischenstufe 4: 3-(3-Amino-3-methyl-butyl)-3H-benzooxazol-2-on

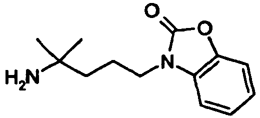

### a) 1-lod-4-methyl-nitro-pentan

Zu 26.0 g (177 mmol) 1-Methyl-4-nitro-pentan-1-ol und 52.8 g (352 mmol) Natriumiodid in 350 mL Acetonitril wird eine Lösung aus 44.7 mL (352 mmol) Chlortrimethylsilan und 50 mL Acetonitril getropft. Anschließend erwärmt man 4 Stunden auf 50°C, destilliert dann das Lösungsmittel ab und versetzt den Rückstand mit 500 mL Diethylether. Es wird nacheinander mit Wasser, Natriumthiosulfat-Lösung und Natriumchlorid-Lösung gewaschen. Die organische Phase wird mit Natriumsulfat getrocknet und eingeengt 34.2 g rotes Öl.

### b) 3-(3-Methyl-3-nitro-butyl)-3H-benzooxazol-2-on

Zu einer Lösung von 4.50 g (33.3 mmol) Benzooxazol-2-on in 50 mL DMF werden portionsweise 1.70 g (42.5 mmol) Natriumhydrid (60%ig) gegeben, wobei die Temperatur durch Kühlung unter 0°C gehalten wird. Nach einer Stunde Rühren wird eine Lösung aus 9.61 g (37.4 mmol) 1-lod-4-methyl-4-nitro-pentan in 20 mL DMF so zugetropft, dass die Temperatur nicht über 5°C steigt. Man läßt über Nacht bei Raumtemperatur Rühren und destilliert das Lösungsmittel ab. Der Rückstand wird in Ethylacetat aufgenommen und nacheinander mit Wasser und Natriumchlorid-Lösung gewaschen, mit Natriumsulfat getrocknet und eingeengt. Es werden 11.0 g Öl erhalten. Massenspektroskopie: [M+H]⁺ = 265.

### c) 3-(3-Amino-3-methyl-butyl)-3H-benzooxazol-2-on

11.0 g 3-(3-Methyl-3-nitro-butyl)-3H-benzooxazol-2-on aus der vorstehend beschriebenen Umsetzung werden in 130 mL Ethanol gelöst und mit Raney-Nickel als Katalysator bei 5 bar über 20 Stunden hydriert. Der Katalysator wird abfiltriert und das Filtrat vom Lösungsmittel befreit. Man versetzt mit 10%iger ethanolische Salzsäure, destilliert das Lösungsmittel ab und verrührt den Rückstand in einem Aceton/Diethylether-Gemisch. Weißer Feststoff. Ausbeute: 6.0 g (77% über 2 Stufen, Hydrochlorid); Schmelzbereich = 145-147°C.

### Zwischenstufe 5: 3-(3-Amino-3-methyl-butyl)-3H-benzooxazol-2-on

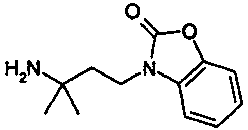

### a) [1,1-Dimethyl-3-(2-oxo-benzooxazol-3-yl)-propyl]-carbaminsäure tert-butyl ester

4.0 g (29.6 mmol) Benzooxazol-2-on werden in 40 mL DMPU gelöst und mit einem Eisbad gekühlt. Unter Schutzgas werden zu dieser Lösung 897 mg (95%ig; 35.5 mmol) Natriumhydrid portionsweise gegeben. Die Reaktionsmischung wird auf Raumtemperatur erwärmt und dann noch eine Stunde gerührt. Es werden 9.85 g (44.4 mmol) (3-Amino-1,1-dimethyl-propyl)-carbaminsäure tert-butylester und 1.97 g (5.3 mmol) Tetrabutylammoniumiodid zugeben und man läßt über Nacht rühren. Durch vorsichtige Zugabe von Natriumhydogencarbonat-Lösung wird die Reaktion beendet. Man versetzt mit Ethylacetat, trennt die wässrige Phase ab und extrahiert diese wiederholt mit Ethylacetat. Die vereinigten organischen Phasen werden mit Natriumchlorid-Lösung gewaschen, mit Natriumsulfat getrocknet und eingeengt. Eine säulenchromatographische Reinigung (Kieselgel; Petrolether/Ethylacetat = 7:3) des Rückstands liefert das gewünschte Produkt in Form eines Öls. Ausbeute 4.1 g (43%); Massenspektroskopie: [M+H]⁺ = 321.

### b) 3-(3-Amino-3-methyl-butyl)-3H-benzooxazol-2-on

Zu einer Lösung 4.0 g (12.5 mmol) [1,1-Dimethyl-3-(2-oxo-benzooxazol-3-yl)-propyl]-carbaminsäure tert-butyl ester in 110 mL Dichlormethan werden bei Raumtemperatur 18 mL Trifluoressigsäure getropft. Man läßt über Nacht Rühren und destilliert dann das Lösungsmittel ab. Das zurückbleibende Öl wird in Diethylether verrührt, wobei ein Feststoff ausfällt, der abfiltriert wird. Nach erneutem Verrühren mit Diethylether und Filtration wird ein beigefarbener Feststoff erhalten. Ausbeute: 3.63 g (65%; Trifluoracetat); Massenspektroskopie: [M+H]⁺ = 221.

### Zwischenstufe 6: 5-Benzyloxy-7-(2-ethoxy-2-hydroxy-acetyl)-3H-benzooxazol-2-on

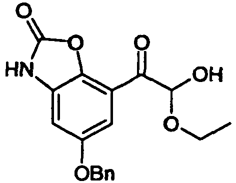

### a) 1-(5-Benzyloxy-2-hydroxy-3-nitro-phenyl)-ethanon

Zu einer Lösung von 81.5 g (0.34 mol) 1-(5-Benzyloxy-2-hydroxy-phenyl)-ethanon (bekannt aus US 4,460581) in 700 mL Essigsäure werden unter Kühlung mit dem Eisbad 18 mL rauchende Salpetersäure so zugetropft, dass die Temperatur nicht über 20°C steigt. Anschließend wird die Reaktionsmischung zwei Stunden bei Raumtemperatur gerührt, auf Eiswasser gegossen und filtriert. Das Produkt wird in Isopropanol umkristallisiert, abgesaugt und mit Isopropanol und Diisopropylether gewaschen. Ausbeute: 69.6 g (72%); Massenspektroskopie [M+H]⁺ = 288.

### b) 1-(3-Amino-5-benzyloxy-2-hydroxy-phenyl)-ethanon

69.5 g (242 mmol) 1-(5-Benzyloxy-2-hydroxy-3-nitro-phenyl)-ethanon werden in 1.4 L Methanol gelöst und in Gegenwart von 14 g Rhodium auf Kohle (10%ig) als Katalysator bei 3 bar und Raumtemperatur hydriert. Anschließend wird der Katalysator abfiltriert und das Filtrat eingeengt. Der Rückstand wird ohne zusätzliche Aufreinigung weiter umgesetzt. Ausbeute: 60.0 g (96%), R_{f}-Wert = 0.45 (Dichlormethan auf Kieselgel).

### c) 7-Acetyl-5-benzyloxy-3H-benzooxazol-2-on

In eine Lösung von 121 g (0.47 mol) 1-(3-Amino-5-benzyloxy-2-hydroxy-phenyl)-ethanon in 800 mL Pyridin werden bei 20 bis 40°C 52 g (0.53 mol) Phosgen eingeleitet. Die Reaktionsmischung wird für 2 Stunden auf 50°C erwärmt, dann auf Eis gegossen und mit konz. Salzsäure angesäuert. Es wird ein rotbrauner Feststoff isoliert, der wiederholt in Ethanol unter Zusatz von Aktivkohle umkristallisiert wird. Ausbeute: 67.5 g (50.6 %); Schmelzbereich: 163-166°C.

### d) 5-Benzyloxy-7-(2-ethoxy-2-hydroxy-acetyl)-3H-benzooxazol-2-on

20 g (71 mmol) 7-Acetyl-5-benzyloxy-3H-benzooxazol-2-on und 8 g (72 mmol) Selendioxid werden in Gegenwart von Aktivkohle in 100 mL Dioxan und 3.1 mL Wasser über 8 Stunden unter Rückfluß gerührt. Der Feststoff wird abfiltriert, das Lösungsmittel abdestilliert und der Rückstand mit 50 mL Ethanol versetzt. Man lässt 15 Minuten refluxieren und filtriert dann über Aktivkohle. Der beim Abkühlen ausfallende Feststoff wird nach 3 Stunden abgesaugt und mit Ethanol und Diethylether gewaschen. Ausbeute: 7 g (29%); Schmelzbereich: 140-143°C.

### Zwischenstufe 7: 6-Benzyloxy-8-(2-ethoxy-2-hydroxy-acetyl)-2,2-dimethyl-4H-benzo[1,4]oxazin-3-on

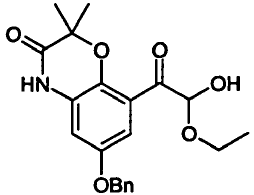

### a) N-(3-Acetyl-5-benzyloxy-2-hydroxy-phenyl)-2-brom-2-methyl-propionamid

Zu einer Lösung von 5.15 g (20 mmol) 1-(3-Amino-5-benzyloxy-2-hydroxy-phenyl)-ethanon in 20 mL Pyridin werden bei 5 bis 20°C 4.64 g (25 mmol) 2-Brom-2-methyl-propionylchlorid getropft. Nach beendeter Zugabe wird 15 Minuten gerührt, mit Eiswasser und 100 mL Ethylacetat versetzt und mit konz. Salzsäure angesäuert. Die organische Phase wird abgetrennt, mit Wasser gewaschen und mit Natriumsulfat getrocknet Nach dem Abdestillieren des Lösungsmittels wird der Rückstand in einem Diethylether/Petrolether-Gemisch auskristallisiert. Ausbeute: 6.8 g (84%); Schmelzbereich: 88-90°C.

### b) 8-Acetyl-6-benzyloxy-2,2-dimethyl-4H-benzo[1,4]oxazin-3-on

6.60 g (16.2 mmol) N-(3-Acetyl-5-benzyloxy-2-hydroxy-phenyl)-2-brom-2-methyl-propionamid und 2.76 g (20 mmol) Kaliumcarbonat werden 1 Stunde in 70 mL Acetonitril unter Rückfluß gerührt. Der Feststoff wird abgesaugt, das Filtrat eingeengt und der Rückstand mit 30 mL Ethylacetat versetzt. Nach erneuter Filtration und dem Abdestillieren des Lösungsmittels wird das Rohprodukt in wenig Methanol auskristallisiert. Ausbeute: 1.00 g (19%); Massenspektroskopie [M+H]⁺ = 326; Schmelzbereich: 148-150°C.

### c) 6-Benzyloxy-8-(2-ethoxy-2-hydroxy-acetyl)-2,2-dimethyl-4H-benzo[1,4]oxazin-3-on

Die Darstellung erfolgt in Analogie zu dem für die Zwischenstufe 6d beschriebenen Verfahren aus 8-Acetyl-6-benzyloxy-2,2-dimethyl-4H-benzo[1,4]oxazin-3-on.

### Zwischenstufe 8: 7-Benzyloxy-5-oxiranyl-1H-chinolin-2-on

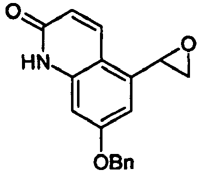

### a) Trifluormethansulfonsäure-2-acetyl-4-benzyloxy-6-nitro-phenylester

92.7 mL (660 mmol) Triethylamin werden zu 90 g (313 mmol) 1-(5-Benzyloxy-2-hydroxy-3-nitro-phenyl)-ethanon in 940 mL Dichlormethan bei -10°C gegeben. Anschließend wird langsam eine Lösung aus 65 mL (394 mmol) Trifluormethansulfonsäureanhydrid und 40 mL Dichlormethan zugetropft. Nach 15 Minuten Rühren bei -5°C wird die Reaktion durch vorsichtige Zugabe von 400 mL Ammoniumchlorid-Lösung und 400 mL Natriumhydrogencarbonat-Lösung beendet. Die organische Phase wird abgetrennt, mit Natriumsulfat getrocknet und eingeengt. Der Rückstand wird in 150 mL Diethylether gelöst und dann durch Zugabe von 800 mL Hexan ausgefällt. Der Feststoff wird abfiltriert, in einem Diethylether/Hexan-Gemisch suspendiert und erneut abgesaugt. Ausbeute: 118 g (90%); Massenspektroskopie: [M+H]⁺ = 420.

### b) 3-(2-Acetyl-4-benzyloxy-6-nitro-phenyl)-acrylsäuremethylester

Zu einer Lösung aus 100 g (238 mmol) Trifluormethansulfonsäure-2-acetyl-4-benzyloxy-6-nitro-phenylester in 360 mL Dioxan werden 5.88 g (6.42 mmol) Tris-(dibenzylidenaceton)-dipalladium, 3.50 g (12.01 mmol) Tri-tert-butylphosphonium tetrafluoroborat, 81.2 mL (371 mmol) Dicyclohexylmethylamin, 105.8 g (286 mmol) Tetrabutylammoniumiodid und 32.6 mL (362 mmol) Methylarcrylat zugegeben. Die Reaktionsmischung wird 2 Stunden bei 80°C unter Stickstoffatmosphäre in Gegenwart von 100 g Molekularsieb 4A gerührt und dann mit 2 L Diethylether und 500 g Kieselgel versetzt. Nach 10 Minuten wird das Kieselgel abgesaugt, wobei wiederholt mit Diethylether nachgewaschen wird. Die vereinigten organischen Phasen werden nacheinander mit 1 N Salzsäure, Natriumcarbonat-Lösung und Natriumchlorid-Lösung gewaschen. Das Lösungsmittel wird abdestilliert, der Rückstand in Ethanol auskristallisiert und der Feststoff abfiltriert und mit Ethanol gewaschen. Ausbeute: 32.2 g (38%); Massenspektroskopie: [M+H]⁺ = 356.

### c) 5-Acetyl-7-benzyloxy-3,4-dihvdro-1H-chinolin-2-on

5.0 g (14.07 mmol) 3-(2-Acetyl-4-benzyloxy-6-nitro-phenyl)-acrylsäuremethylester werden mit 100 mL Ethanol versetzt und mit Raney-Nickel als Katalysator bei 4 bar hydriert. Der Katalysator wird abgetrennt und das Filtrat mit 15 mL 2 N Salzsäure angesäuert. Das auskristallisierende Produkt wird abgesaugt und getrocknet. Ausbeute: 1.0 g (24%); Massenspektroskopie: [M+H]⁺ = 296.

### d) 5-Acetyl-7-benzyloxy-1H-chinolin-2-on

13.0 g (44 mmol) 5-Acetyl-7-benzyloxy-3,4-dihydro-1H-chinolin-2-on werden in 130 mL Dioxan suspendiert und mit 15.0 g (66 mmol) 2,3-Dichlor-5,6-dicyanobenzochinon versetzt. Man lässt 30 Minuten Refluxieren, kühlt auf Raumtemperatur ab und rührt weitere 2 Stunden. Der Feststoff wird abfiltriert, mit Dioxan gewaschen und in 600 mL Dichlormethan/Methanol (9:1) gelöst. Die Lösung wird mit Natriumhydrogencarbonat-Lösung gewaschen, mit Natriumsulfat getrocknet und eingeengt. Anschließend wird der Rückstand in Methanol suspendiert, filtriert und getrocknet. Ausbeute: 8.3 g (64%); Massenspektroskopie: [M+H]⁺ = 294.

### e) 7-Benzyloxy-5-(2-chlor-acetyl)-1H-chinolin-2-on

7.0 g (23.9 mmol) 5-Acetyl-7-benzyloxy-1H-chinolin-2-on und 19.0 g (54.6 mmol) Benzyltrimethylammonium dichloriodat werden in 43 mL Essigsäure, 7 mL Wasser und 147 mL Dichlorethan bei 65°C gerührt. Nach 4.5 Stunden wird die Reaktion durch Zugabe von 400 mL Natriumcarbonat-Lösung und 50 mL 5%iger Natriumsulfit-Lösung beendet. Die unlöslichen Bestandteile werden abgesaugt, mit Wasser gewaschen und getrocknet. Ausbeute: 6.0 g (77%); Massenspektroskopie: [M+H]⁺ = 328.

### f) 7-Benzyloxy-5-oxiranyl-1H-chinolin-2-on

6.0 g (18.3 mmol) 7-Benzyloxy-5-(2-chlor-acetyl)-1H-chinolin-2-on werden in 150 mL Tetrahydrofuran vorgelegt und bei 0 bis 5°C mit 434 mg (19.9 mmol) Lithiumborhydrid versetzt. Es wird 30 Minuten gerührt, dann werden 43 mL einer 2.5 molare Natriumhydroxid-Lösung zugegeben und man lässt weitere 4 Stunden unter Erwärmung auf Raumtemperatur Rühren. Die Mischung wird mit Natriumchlorid-Lösung versetzt, filtriert und wiederholt mit Ethylacetat/Tetrahydrofuran (1:1) extrahiert. Der abfiltrierte Feststoff und die organischen Phasen werden vereinigt und vom Lösungsmittel befreit. Der Rückstand wird in Methanol suspendiert, abgesaugt und getrocknet. Ausbeute 4.8 g (89%); Massenspektroskopie: [M+H]⁺ = 294.

### Zwischenstufe 9: 1-(3-Amino-3-methyl-butyl)-4-methoxy-1,3-dihydro-benzoimidazol-2-on

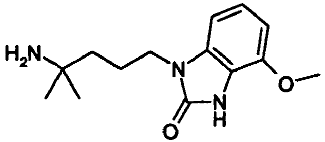

### a) 4-Methyl-4-nitro-pentan-1-ol

50 g (0.285 mol) 4-Methyl-4-nitro-pentansäuremethylester werden in einer 5:4 Mischung von THF/Ethanol (1000 mL) gelöst. Die Lösung wird auf -10°C gekühlt und mit 24.2 g (0.571 mol) Lithiumchlorid versetzt. Anschließend werden 21.6 g (0.571 mol) Lithiumborhydrid portionsweise zugegeben. Es wird 30 Minuten bei -10°C gerührt und dann über Nacht auf Raumtemperatur erwärmt. Die Reaktionsmischung wird 6 Stunden bei 60°C und über Nacht bei Raumtemperatur gerührt. Es wird mit Wasser versetzt und mit verdünnter Salzsäure auf pH 6 eingestellt Das Lösungsmittel wird abdestilliert und der Rückstand mit Wasser versetzt. Man extrahiert mit Dichlormethan, wäscht die vereinigten organischen Phasen mit Wasser und Ammoniumchlorid-Lösung und trocknet mit Natriumsulfat. Nach dem Entfernen des Lösungsmittels wird das Produkt als gelbes Öl erhalten. Ausbeute: 40.0 g (95%); Massenspektroskopie: [M+H]⁺ = 148.

### b) 1-lod-4-methyl-4-nitro-pentan

Zu 40 g (0.272 mol) 4-Methyl-4-nitro-pentan-1-ol und 81.5 g (0.544 mol) Natriumiodid in 350 mL Acetonitril werden 70 mL (0.544 mol) Trimethylchlorsilan bei Raumtemperatur getropft. Die Reaktionsmischung wird filtriert, eingeengt und mit Diethylether versetzt. Die organische Phase wird mit Natriumbisulfit-Lösung und Wasser gewaschen, getrocknet und vom Lösungsmittel befreit. Gelbes Öl. Ausbeute: 56.0 g (80%); Massenspektroskopie: [M-NO₂]⁺ = 211.

### c) 2-Methoxy-6-nitro-phenylamin

Zu einer Lösung von 25 g (0.162 mol) 2-Amino-3-nitro-phenol in 200 mL DMF wird 85%ige Kaliumhydroxid-Lösung gegeben (11.7 g, 0.179 mol). Anschließend werden 11.1 mL (0.178 mol) lodmethan zugetropft und man lässt über Nacht bei Raumtemperatur rühren. Die Reaktionsmischung wird auf Eis gegossen und eine Stunde gerührt. Das ausgefallene Produkt wird abfiltriert, mit Wasser gewaschen und getrocknet. Ausbeute: 23.8 g (87%); Massenspektroskopie: [M+H]⁺ = 169.

### d) (2-Methoxy-6-nitro-phenyl)-carbaminsäureethylester

Unter Rückfluß werden zu einer Lösung von 23.8 g (0.142 mol) 2-Methoxy-6-nitro-phenylamin in 300 mL THF 17.1 mL (0.141 mol) Trichlormethylchlorformiat getropft und anschließend wird 4 Stunden bei dieser Temperatur gerührt. Das Lösungsmittel wird abdestilliert und der Rückstand mit Isopropanol verrührt, wobei ein gelber Feststoff ausfällt. Ausbeute: 25.0 g (73%); Massenspektroskopie: [M+H]⁺ = 241.

### e) (2-Amino-6-methoxy-phenyl)-carbaminsäureethylester

25.0 g (0.104 mol) (2-Methoxy-6-nitro-phenyl)-carbaminsäureethylester werden in 400 mL Methanol gelöst. 116.4 g (0.516 mol) SnCl₂ 2H₂O werden zugeben und man lässt 3 Stunden refluxieren. Die Reaktionsmischung wird eingeengt, mit Natriumcarbonat-Lösung versetzt und filtriert. Die wässrige Phase wird wiederholt mit Dichlormethan extrahiert und die vereinigten organischen Phasen werden mit Natriumchlorid-Lösung gewaschen, getrocknet und eingeengt. Der beim Stehen auskristallisierende Rückstand wird mit Isopropanol verrührt. Ausbeute: 13.0 g (59%); Massenspektroskopie: [M+H]⁺ = 211.

### f) 7-Methoxy-2-oxo-2,3-dihydro-benzoimidazol-1-carbonsäureethylester

13.0 g (0.062 mol) (2-Amino-6-methoxy-phenyl)-carbaminsäureethylester und 10.3 mL (0.074 mol) Triethylamin in 100 mL Dichlormethan werden unter Eiskühlung zu einer Lösung von 8.20 mL (0.068 mol) Trichlormethylchlorformiat in 50 mL Dichlormethan gegeben. Nach 4 Stunden Rühren bei Raumtemperatur wird die Reaktionsmischung auf Eis gegossen und mit Dichlormethan extrahiert. Die vereinigten organischen Phasen werden mit Wasser gewaschen, getrocknet und vom Lösungsmittel befreit. Der Rückstand wird in Diethylether verrührt. Ausbeute: 9.0 g (62%); Massenspektroskopie: [M+H]⁺ = 237.

### g) 4-Methoxy-1-(3-methyl-3-nitro-butyl)-1,3-dihydro-benzoimidazol-2-on

4.0 g (17 mmol) 7-Methoxy-2-oxo-2,3-dihydro-benzoimidazol-1-carbonsäureethylester in DMF werden unter Kühlung mit dem Eisbad mit 85%iger Kaliumhydroxid-Lösung (3.3 g, 51 mmol) versetzt. Nach 30 Minuten wird eine Lösung von 5.2 g (21 mmol) 1-lod-4-methyl-4-nitro-pentan in DMF zugegeben und es wird über Nacht bei Raumtemperatur gerührt. Die Reaktionsmischung wird mit Wasser verdünnt und mit Ethylacetat extrahiert. Die vereinigten organischen Phasen werden mit Wasser gewaschen, getrocknet und vom Lösungsmittel befreit. Das zurückbleibende Öl wird mittels Chromatographie an einer Kieselgelsäule gereinigt (Cyclohexan/Ethylacetat-Gradient). Ausbeute: 0.5 g (8%); Massenspektroskopie: [M+H]⁺ = 366.

### h) 1-(3-Amino-3-methyl-butyl)-4-methoxy-1,3-dihydro-benzoimidazol-2-on

1.4 g (4.8 mmol) 4-Methoxy-1-(3-methyl-3-nitro-butyl)-1,3-dihydro-benzoimidazol-2-on werden in Methanol gelöst und in Gegenwart von Raney-Nickel bei 3 bar hydriert. Der Katalysator wird abgetrennt, das Lösungsmittel abdestilliert und der Rückstand wird in ethanolischer Salzsäure gelöst. Die Lösungsmittel werden destillativ entfernt und der zurückbleibende Feststoff wird mit Isopropanol verrührt. Ausbeute: 0.6 g (42%, Hydrochlorid); Massenspektroskopie: [M+H]⁺ = 300.

### Zwischenstufe 10: 1-(3-Amino-3-methyl-butyl)-5-methoxy-3-methyl-1,3-dihydrobenzoimidazol-2-on

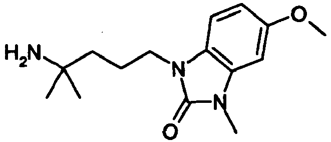

### a) (5-Methoxy-2-nitro-phenyl)-methyl-amin

Zu 14.3 g (83.56 mmol) 3-Fluor-4-nitro-anisol und 12.71 g (92.02 mmol) Kaliumcarbonat in 200 mL Dichlormethan werden 83.5 mL (167.0 mmol) einer 2 molaren Lösung von Methylamin in THF getropft. Man lässt über Nacht rühren und versetzt dann mit Wasser. Die organische Phase wird nacheinander mit Wasser und Ammoniumchlorid-Lösung gewaschen, getrocknet und eingeengt Der zurückbleibende gelbe Feststoff wird mit Hexan verrührt. Ausbeute: 12.7 g (84%); Massenspektroskopie: [M+H]⁺ = 183.

### b) 4-Methoxy-N-2-methyl-benzol-1,2-diamin

12.5 g (68.6 mmol) (5-Methoxy-2-nitro-phenyl)-methyl-amin und 77.39 g (343.0 mmol) SnCl₂ 2H₂O in 200 mL Ethanol werden über 6 Stunden unter Rückfluß erwärmt. Die Reaktionsmischung wird mit Natriumcarbonat-Lösung gewaschen, filtriert und eingeengt. Der Rückstand wird mit Wasser versetzt und mit Ethylacetat extrahiert. Die vereinigten organischen Phasen werden mit Wasser gewaschen, getrocknet und vom Lösungsmittel befreit. Öl. Ausbeute: 8.0 g (77%); Massenspektroskopie: [M+H]⁺ = 153.

### c) 5-Methoxy-1-methyl-1,3-dihydro-benzoimidazol-2-on

8.0 g (52.56 mmol) 4-Methoxy-N-2-methyl-benzol-1,2-diamin and 8.7 mL (63.00 mmol) Triethylamin werden in 100 mL Dichlormethan gelöst und zu 7 mL (58.00 mmol) Trichlormethylchlorformiat in 50 mL Dichlormethan getropft. Die Reaktionsmischung wird über Nacht bei Raumtemperatur gerührt, anschließend in Eiswasser gegossen und mit Dichlormethan extrahiert. Die vereinigten organischen Phasen werden mit Wasser gewaschen, getrocknet und eingeengt. Der zurückbleibende Feststoff wird mit Diethylether verrührt. Ausbeute: 4.2 g (45%); Massenspektroskopie: [M+H]⁺ = 179.

### d) 5-Methoxy-3-methyl-1-(3-methyl-3-nitro-butyl)-1,3-dihydro-benzoimidazol-2-on

Zu 2.5 g (14 mmol) 5-Methoxy-1-methyl-1,3-dihydro-benzoimidazol-2-on in 30 mL DMF werden unter Kühlung mit dem Eisbad 1.1 g (28 mmol) 60%iges Natriumhydrid gegeben. Nach 30 Minuten wird eine Lösung aus 1-lod-4-methyl-4-nitro-pentan in 20 mL DMF eingeleitet und man lässt über Nacht Rühren. Es wird mit Wasser verdünnt und mit Ethylacetat extrahiert. Die vereinigten organischen Phasen werden mit Wasser gewaschen, getrocknet und eingeengt Der zurückbleibende Feststoff wird mit Diethyletheer verrührt. Ausbeute: 2.7 g (63%); Massenspektroskopie: [M+H]⁺ = 308.

### e) 1-(3-Amino-3-methyl-butyl)-5-methoxy-3-methyl-1,3-dihydro-benzoimidazol-2-on

2.7 g (8.7 mmol) 5-Methoxy-3-methyl-1-(3-methyl-3-nitro-butyl)-1,3-dihydro-benzoimidazol-2-on und 9.93 g (44.0 mmol) SnCl₂ 2H₂O in 200 mL Ethanol werden 3 Stunden refluxiert. Die Reaktionsmischung wird eingeengt, mit Natriumcarbonat-Lösung versetzt und filtriert. Das Filtrat wird mit Ethylacetat extrahiert und die vereinigten organischen Phasen werden mit Wasser gewaschen, getrocknet und vom Lösungsmittel befreit. Der Rückstand wird in Ethanol gelöst und die Lösung mit etherischer Salzsäure versetzt. Nach dem Abdestillieren des Lösungsmittel wird der zurückbleibende Feststoff mit Diisopropylether verrührt. Ausbeute: 0.7 g (29%); Massenspektroskopie: [M+H]⁺ = 278.

### Zwischenstufe 11: 3-(4-Amino-4-methyl-pentyl)-5-fluor-1-methyl-1,3-dihydro-benzoimidazol-2-on

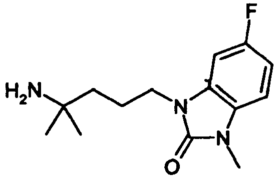

### a) (4-Fluor-2-nitro-phenyl)-methyl-amin

Zu 25 g (157 mmol) 2,4-Difluor-nitrobenzol und 23.9 g (173 mmol) Kaliumcarbonat in 300 mL Dichlormethan werden unter Kühlung 157 ml (314 mmol) einer 2 molaren Lösung von Methylamin in THF getropft. Es wird über Nacht bei Raumtemperatur gerührt und dann mit Wasser versetzt. Die organische Phase wird mit Wasser gewaschen, getrocknet und eingeengt. Der Rückstand wird mit Diethylether verrührt. Ausbeute: 18 g (69%); Massenspektroskopie [M+H]⁺ = 171.

### b) 4-Fluor-N-1-methyl-benzol-1,2-diamin

22 g (0.12 mol) (4-Fluor-2-nitro-phenyl)-methyl-amin in 250 mL Ethanol werden mit Palladium auf Kohle als Katalysator bei 4 bar Wasserstoffdruck hydriert. Der Katalysator wird abgetrennt und das Lösungsmittel abdestilliert. Das zurückbleibende Öl wird mittels Chromatographie gereinigt (Kieselgel, Hexan/Ethylacetat-Gradient). Das Produkt wird in Form eines Öls erhalten. Ausbeute: 9 g (50%); Massenspektroskopie [M+H]⁺= 141.

### c) 5-Fluor-1-methyl-1,3-dihydro-benzoimidazol-2-on

13.0 g (92.1 mmol) 4-Fluor-N-1-methyl-benzol-1,2-diamin werden in Analogie zu dem für die Zwischenstufe 10c beschriebenen Verfahren mit Trichlormethylchlorformiat umgesetzt. Nach Verrühren in Diethylether wird das Produkt als Feststoff isoliert. Ausbeute: 6.0 g (39%); Massenspektroskopie: [M+H]⁺ = 167.

### d) 5-Fluor-1-methyl-3-(4-methyl-4-nitro-pentyl)-1,3-dihydro-benzoimidazol-2-on

Zu einer Lösung von 2.1 g (12.6 mmol) 5-Fluor-1-methyl-1,3-dihydro-benzoimidazol-2-on in DMF werden zunächst 0.624 g (13.9 mmol) 60%iges Natriumhydrid und dann unter Kühlung 4.6 g (17.8 mmol) 1-lod-4-methyl-4-nitro-pentan in 10 mL DMF gegeben. Die Reaktionsmischung wird über Nacht bei Raumtemperatur gerührt, dann auf Wasser gegossen und mit Diethylether extrahiert. Die organischen Phasen werden eingeengt und der Rückstand in Isopropylether umkristallisiert. Ausbeute: 1.8 g (48%); Massenspektroskopie [M+H]⁺ = 296.

### e) 3-(4-Amino-4-methyl-pentyl)-5-fluor-1-methyl-1,3-dihydro-benzoimidazol-2-on

1.8 g (6.09 mmol) 5-Fluor-1-methyl-3-(4-methyl-4-nitro-pentyl)-1,3-dihydro-benzoimidazol-2-on in 50 mL Methanol werden mit Raney-Nickel als Katalysator bei 3 bar Wasserstoffdruck hydriert. Der Katalysator wird abgetrennt und das Lösungsmittel abdestilliert. Zur Herstellung des Hydrochlorids wird der Rückstand mit Ethanol und Salzsäure in Diethylether versetzt. Ausbeute: 1.5 g (83%, Hydrochlorid); Schmelzbereich = 225-228°C; Massenspektroskopie [M+H]⁺ = 303.

### Zwischenstufe 12: 3-(4-Amino-4-methyl-pentyl)-4-fluoro-1-methyl-1,3-dihydro-benzoimidazol-2-on

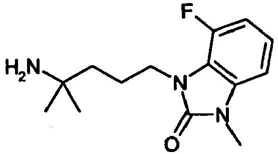

### a) (3-Fluor-2-nitro-phenyl)-methyl-amin

Umsetzung von 2.0 g (2.6 mmol) 2,6-Difluor-nitrobenzol mit einer 2 molaren Lösung von Methylamin in THF analog dem Verfahren zur Herstellung von Zwischenstufe 10a. Roter Feststoff. Ausbeute: 1.8 g (86%); Massenspektroskopie: [M+H]⁺ = 171.

### b) 3-Fluor-N-1-methyl-benzene-1,2-diamin

Reduktion von 8.0 g (47.0 mmol) (3-Fluor-2-nitro-phenyl)-methyl-amin mit SnCl₂ x 2H₂O nach dem für die Zwischenstufe 10b beschriebenen Verfahren. Rotes Öl. Ausbeute: 4.5 g (68%); Massenspektroskopie: [M+H]⁺ = 141.

### c) 4-Fluor-1-methyl-1,3-dihydro-benzoimidazol-2-on

Hergestellt aus 4.5 g (32.1 mmol) 3-Fluor-N-1-methyl-benzol-1,2-diamin analog der für die Zwischenstufe 10c beschriebenen Weise. Brauner Feststoff. Ausbeute: 1.4 g (26%); Massenspektroskopie: [M+H]⁺ = 167.

### d) 4-Fluor-1-methyl-3-(4-methyl-4-nitro-pentyl)-1,3-dihydro-benzoimidazol-2-on

Herstellung aus 1.4 g (8.42 mmol) 4-Fluor-1-methyl-1,3-dihydro-benzoimidazol-2-on analog dem für die Zwischenstufe 10d beschriebenen Verfahren. Gelbes Öl. Ausbeute: 1.7 g (68%); Massenspektroskopie: [M+H]⁺ = 296.

### e) 3-(4-Amino-4-methyl-pentyl)-4-fluor-1-methyl-1,3-dihydro-benzoimidazol-2-on

Eine Lösung von 2 g (6.7 mmol) 4-Fluor-1-methyl-3-(4-methyl-4-nitro-pentyl)-1,3-dihydro-benzoimidazol-2-on in Methanol wird in Gegenwart von Raney-Nickel bei 3 bar Wasserstoffdruck hydriert. Nach Abtrennung des Katalysators wird Salzsäure in Diethylether zugesetzt. Das ausfallende Hydrochlorid wird abfiltriert und getrocknet. Ausbeute: 1.5 g (83%, Hydrochlorid); Schmelzbereich = 230-232°C; Massenspektroskopie: [M+H]⁺ = 303.

### SYNTHESE VON ENDVERBINDUNGEN

### Allgemeine Arbeitsvorschrift 1:

1 mmol Glyoxalaldehyd oder -acetal und 1 mmol Amin werden 30 Minuten in 5 mL Tetrahydrofuran bei 50°C gerührt. Man kühlt auf 0°C ab und tropft unter Argonatmosphäre 1.5 mL einer 2 molaren Lösung von Lithiumborhydrid in Tetrahydrofuran zu. Es wird 30 min bei 0°C gerührt, mit 10 mL Dichlormethan und 3 mL Wasser versetzt, eine weitere Stunde bei Raumtemperatur gerührt und dann über Kieselgur filtriert, wobei man mit Dichlormethan eluiert. Das Eluat wird vom Lösungsmittel befreit und der Rückstand, falls notwendig, chromatographisch gereinigt. Der so erhaltene Benzylether wird in Methanol gelöst und mit Palladium auf Kohle (10%ig) als Katalysator bei 2.5 bar und Raumtemperatur hydriert. Anschließend wird der Katalysator abgetrennt und das Rohprodukt mittels Chromatographie (Reverse Phase, Acetonitril/Wasser-Gradient mit 0.1% Trifluoressigsäure) gereinigt oder in Acetonitril auskristallisiert.

### Allgemeine Arbeitsvorschrift 2

1 mmol Glyoxalaldehyd oder -acetal und 1 mmol Amin werden in 5 mL Ethanol suspendiert und auf 70°C erwärmt. Die entstandene Lösung wird eine Stunde bei 70°C gerührt und dann auf Raumtemperatur abgekühlt. Nach Zugabe von 113 mg (3 mmol) Natriumborhydrid wird 3 Stunden bei Raumtemperatur gerührt, mit 0.7 mL gesättigter Kaliumcarbonatlösung versetzt und weitere 30 Minuten gerührt. Es wird über Aluminiumoxid (basisch) filtriert, wiederholt mit Dichlormethan/Methanol 15:1 nachgewaschen, eingeengt und chromatographiert (Kieselgel; Dichlormethan mit 0-10% Methanol:Ammoniak = 9:1). Die so erhaltene Benzylverbindung wird in 10 mL Methanol gelöst und mit Palladium auf Kohle als Katalysator bei 1 bar Wasserstoffdruck hydriert. Anschließend wird der Katalysator abfiltriert und das Filtrat eingeengt.

### Beispiel 1: 8-{2-[1,1-Dimethyl-3-(6-methyl-2-oxo-2,3-dihydro-benzoimidazol-1-yl)-propylamino]-1-hydroxy-ethyl}-6-hydroxy-4H-benzo[1,4]oxazin-3-on

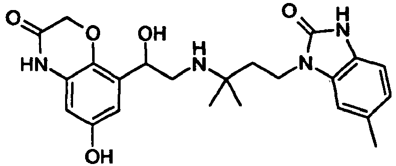

Die Verbindung wird nach der allgemeinen Arbeitsvorschrift 1 aus 357 mg (1 mmol) 6-Benzyloxy-8-(2-ethoxy-1,2-dihydroxy-ethyl)-4H-benzo[1,4]oxazin-3-on und 233 mg (1 mmol) 1-(3-Amino-3-methyl-butyl)-6-methyl-1,3-dihydro-benzoimidazol-2-on hergestellt. Ausbeute: 170 mg (31%, Trifluoracetat); Massenspektroskopie: [M+H]⁺ = 441.

### Beispiel 2: 8-{2-[1,1-Dimethyl-3-(2-oxo-5-trifluormethyl-2,3-dihydro-benzoimidazol-1-yl)-propylamino]-1-hydroxy-ethyl}-6-hydroxy-4H-benzo[1,4]oxazin-3-on

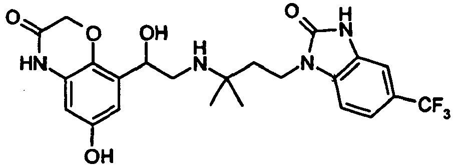

Herstellung nach der allgemeinen Arbeitsvorschrift 1 aus 357 mg (1 mmol) 6-Benzyloxy-8-(2-ethoxy-1,2-dihydroxy-ethyl)-4H-benzo[1,4]oxazin-3-on und 287 mg (1 mmol) 1-(3-Amino-3-methyl-butyl)-5-trifluormethyl-1,3-dihydro-benzoimidazol-2-on. Ausbeute: 76 mg (13%, Trifluoracetat); Massenspektroskopie: [M+H]⁺ = 495.

### Beispiel 3: 8-{2-[1,1-Dimethyl-4-(2-oxo-benzooxazol-3-yl)-butylamino]-1-hydroxy-ethyl}-6-hydroxy-4H-benzo[1,4]oxazin-3-on

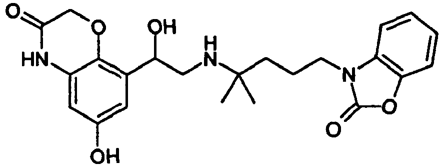

357 mg (1 mmol) 6-Benzyloxy-8-(2-ethoxy-1,2-dihydroxy-ethyl)-4H-benzo[1,4]oxazin-3-on und 287 mg (1 mmol) 3-(4-Amino-4-methyl-pentyl)-3H-benzooxazol-2-on werden nach der allgemeinen Arbeitsvorschrift 1 umgesetzt. Nach hydrogenolytischer Abspaltung der Benzylschutzgruppe wird ein Öl isoliert, aus dem das Produkt durch Verrühren in einem Aceton/Diethylether-Gemisch erhalten wird. Ausbeute: 161 mg (29%, Trifluoracetat); Massenspektroskopie: [M+H]⁺ = 442.

### Beispiel 4: 8-{2-[1,1-Dimethyl-3-(3-methyl-2-oxo-2,3-dihydro-benzoimidazol-1-yl)-propylamino]-1-hydroxy-ethyl}-6-hydroxy-4H-benzo[1,4]oxazin-3-on

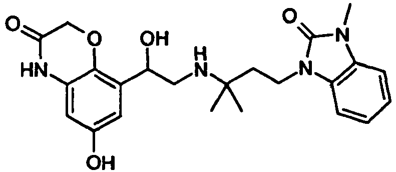

Herstellung nach der allgemeinen Arbeitsvorschrift 2 aus 357 mg (1 mmol) 6-Benzyloxy-8-(2-ethoxy-1,2-dihydroxy-ethyl)-4H-benzo[1,4]oxazin-3-on und 233 mg (1 mmol) 1-(3-Amino-3-methyl-butyl)-3-methyl-1,3-dihydro-benzoimidazol-2-on. Ausbeute: 270 mg (61 %); Massenspektroskopie: [M+H]⁺ = 441.

### Beispiel 5: 8-{2-[1,1-Dimethyl-3-(2-oxo-2,3-dihydro-benzoimidazol-1-yl)-propylamino]-1-hydroxy-ethyl}-6-hydroxy-4H-benzo[1,4]oxazin-3-on

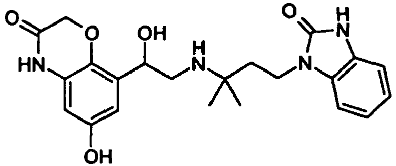

Die Zielverbindung wird nach der allgemeinen Arbeitsvorschrift 2 aus 357 mg (1 mmol) 6-Benzyloxy-8-(2-ethoxy-1,2-dihydroxy-ethyl)-4H-benzo[1,4]oxazin-3-on und 219 mg (1 mmol) 1-(3-Amino-3-methyl-butyl)-1,3-dihydro-benzoimidazol-2-on erhalten. Ausbeute: 187 mg (44%); Massenspektroskopie: [M+H]⁺ = 427.

### Beispiel 6: 8-{2-[1,1-Dimethyl-4-(2-oxo-2,3-dihydro-benzoimidazol-1-yl)-butylamino]-1-hydroxy-ethyl}-6-hydroxy-4H-benzo[1,4]oxazin-3-on

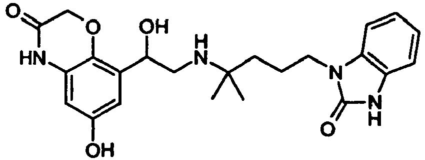

Hergestellt nach der allgemeinen Arbeitsvorschrift 2 aus 357 mg (1 mmol) 6-Benzyloxy-8-(2-ethoxy-1,2-dihydroxy-ethyl)-4H-benzo[1,4]oxazin-3-on und 233 mg (1 mmol) 1-(4-Amino-4-methyl-pentyl)-1,3-dihydro-benzoimidazol-2-on. Ausbeute: 192 mg (44%); Massenspektroskopie: [M+H]⁺ = 441.

### Beispiel 7: 8-{2-[1,1-Dimethyl-3-(2-oxo-benzooxazol-3-yl)-propylamino]-1-hydroxy-ethyl}-6-hydroxy-4H-benzo[1,4]oxazin-3-on

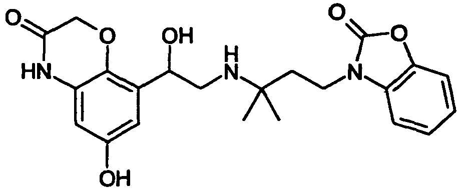

Die Herstellung erfolgt nach der allgemeinen Arbeitsvorschrift 1 aus 357 mg (1 mmol) 6-Benzyloxy-8-(2-ethoxy-1,2-dihydroxy-ethyl)-4H-benzo[1,4]oxazin-3-on und 220 mg (1 mmol) 3-(3-Amino-3-methyl-butyl)-3H-benzooxazol-2-on. Ausbeute: 227 mg (42%, Trifluoracetat); Massenspektroskopie: [M+H]⁺ = 428.

### Beispiel 8: 7-{2-[1,1-Dimethyl-3-(2-oxo-2,3-dihydro-benzoimidazol-1-yl)-propylamino]-1-hydroxy-ethyl}-5-hydroxy-3H-benzooxazol-2-on

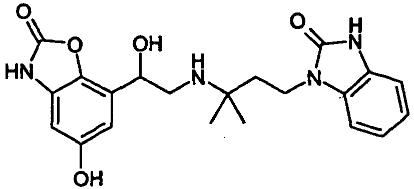

### a) 5-Benzyloxy-7-{2-[1,1-dimethyl-3-(2-oxo-2,3-dihydro-benzoimidazol-1-yl)-propylamino]-1-hydroxy-ethyl}-3H-benzooxazol-2-on

343 mg (1 mmol) 5-Benzyloxy-7-(2-ethoxy-2-hydroxy-acetyl)-3H-benzooxazol-2-on und 219 mg (1 mmol) 1-(3-Amino-3-methyl-butyl)-1,3-dihydro-benzoimidazol-2-on werden in 15 mL Ethanol 1.5 Stunden bei 80°C gerührt. Nach Abkühlung auf Raumtemperatur werden 80 mg (2 mmol) Natriumborhydrid zugesetzt und man lässt 2 Stunden rühren. Die Reaktionsmischung wird mit 3 mL 1 molarer Salzsäure-Lösung angesäuert, 10 Minuten gerührt und mit Kaliumcarbonat-Lösung alkalisch gestellt. Es wird mit Ethylacetat extrahiert, die organischen Phasen werden mit Natriumsulfat getrocknet und das Lösungsmittel wird abdestilliert. Der Rückstand wird chromatographisch über eine Kieselgelsäule gereinigt (Dichlormethan/Methanol-Gradient). Beiger Feststoff. Ausbeute: 340 mg (68%); Massenspektroskopie [M+H]⁺ = 503.

### b) 7-{2-[1,1-Dimethyl-3-(2-oxo-2,3-dihydro-benzoimidazol-1-yl)-propylamino]-1-hydroxyethyl}-5-hydroxy-3H-benzooxazol-2-on

320 mg (0.64 mmol) 5-Benzyloxy-7-{2-[1,1-dimethyl-3-(2-oxo-2,3-dihydro-benzoimidazol-1-yl)-propylamino]-1-hydroxy-ethyl}-3H-benzooxazol-2-on werden in 12 ml Methanol gelöst und mit Palladium auf Kohle als Katalysator bei Raumtemperatur hydriert. Der Katalysator wird abgetrennt und das Filtrat vom Lösungsmittel befreit. Beiger Feststoff. Ausbeute: 150 mg (57%); Massenspektroskopie [M-H]⁺ = 411.

### Beispiel 9: 8-{2-[3-(3-Benzyl-2-oxo-2,3-dihydro-benzoimidazol-1-yl)-1,1-dimethyl-propylamino]-1-hydroxy-ethyl}-6-hydroxy-2,2-dimethyl-4H-benzo[1,4]oxazin-3-on

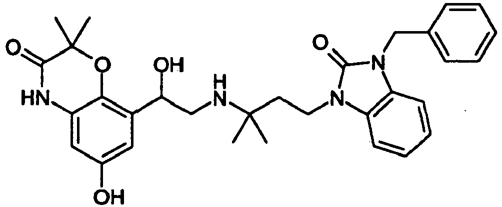

### a) 1-(3-Amino-3-methyl-butyl)-3-benzyl-1,3-dihydro-benzoimidazol-2-on

[1,1-Dimethyl-3-(2-oxo-2,3-dihydro-benzoimidazol-1-yl)-propyl]-carbaminsäure tert-butylester, Benzylchlorid und Kalium-tert-butylat werden über Nacht bei Raumtemperatur in Dimethylsulfoxid gerührt. Das aus der Umsetzung erhaltene Alkylierungsprodukt [3-(3-Benzyl-2-oxo-2,3-dihydro-benzoimidazol-1-yl)-1,1-dimethyl-propyl]-carbaminsäure tert-butylester wird zur Abspaltung der Schutzgruppe anschließend mit Trifluoressigsäure/Dichlormethan behandelt. Massenspektroskopie [M+H]⁺ = 310.

### b) 8-{2-[3-(3-Benzyl-2-oxo-2,3-dihydro-benzoimidazol-1-yl)-1,1-dimethyl-propylamino]-1-hydroxy-ethyl}-6-hydroxy-2,2-dimethyl-4H-benzo[1,4]oxazin-3-on

385 mg (1 mmol) 6-Benzyloxy-8-(2-ethoxy-2-hydroxy-acetyl)-2,2-dimethyl-4H-benzo[1,4]oxazin-3-on und 423 mg (1 mmol) 1-(3-Amino-3-methyl-butyl)-3-benzyl-1,3-dihydro-benzoimidazol-2-on werden nach der allgemeinen Arbeitsvorschrift 1 umgesetzt und aufgearbeitet Ausbeute: 39 mg (6%, Trifluoracetat); Massenspektroskopie [M+H]⁺ = 545.

### Beispiel 10: 8-{2-[3-(3-Cyclopropylmethyl-2-oxo-2,3-dihydro-benzoimidazol-1-yl)-1,1-dimethyl-propylamino]-1-hydroxy-ethyl}-6-hydroxy-4H-benzo[1,4]oxazin-3-on

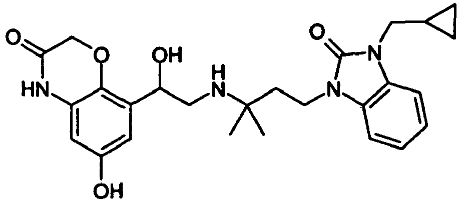

### a) 1-(3-Amino-3-methyl-butyl)-3-cyclopropylmethyl-1,3-dihydro-benzoimidazol-2-on

Die Umsetzung von [1,1-Dimethyl-3-(2-oxo-2,3-dihydro-benzoimidazol-1-yl)-propyl]-carbaminsäure tert-butylester mit (Chlormethyl)-cyclopropan und Kalium-tert-butylat in Dimethylsulfoxid bei Raumtemperatur liefert [3-(3-Cyclopropylmethyl-2-oxo-2,3-dihydro-benzoimidazol-1-yl)-1,1-dimethyl-propyl]-carbaminsäure tert-butylester. Anschließend wird die Schutzgruppe des Alkylierungsproduktes durch Behandlung mit Trifluoressigsäure in Dichlormethan abgespalten. Massenspektroskopie [M+H]⁺ = 274.

### b) 8-{2-[3-(3-Cyclopropylmethyl-2-oxo-2,3-dihydro-benzoimidazol-1-yl)-1,1-dimethyl-propylamino]-1-hydroxy-ethyl}-6-hydroxy-4H-benzo[1,4]oxazin-3-on

165 mg (0.5 mmol) 6-Benzyloxy-8-(2-ethoxy-2-hydroxy-acetyl)-4H-benzo[1,4]oxazin-3-on und 194 mg (0.5 mmol) 1-(3-Amino-3-methyl-butyl)-3-cyclopropylmethyl-1,3-dihydro-benzoimidazol-2-on werden in 8 mL Ethanol gelöst und 1.5 Stunden bei 80°C gerührt. Man lässt auf Raumtemperatur abkühlen, versetzt mit 19 mg (0.5 mmol) Natriumborhydrid und rührt weitere 2 Stunden. Die Reaktionsmischung wird mit 1 molarer Salzsäure angesäuert, 10 Minuten gerührt und mit Kaliumcarbonat-Lösung alkalisch gestellt. Es wird Ethylacetat zugesetzt und die wässrige Phase wird durch Filtration über Kieselgur abgetrennt. Die organische Phase wird vom Lösungsmittel befreit und der Rückstand in Acetonitril/Wasser suspendiert. Die anschließende Debenzylierung erfolgt analog der allgemeinen Arbeitsvorschrift 1. Ausbeute: 77 mg (26%, Trifluoracetat); Massenspektroskopie [M+H]⁺ = 481.

### Beispiel 11: 5-{2-[1,1-Dimethyl-3-(2-oxo-2,3-dihydro-benzoimidazol-1-yl)-propylamino]-1-hydroxy-ethyl}-7-hydroxy-1H-quinolin-2-on

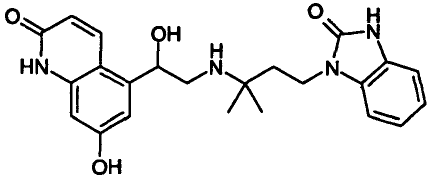

### a) 7-Benzyloxy-5-{[2-[1,1-dimethyl-3-(2-oxo-2,3-dihydro-benzoimidazol-1-yl)-propylamino]-1-hydroxy-ethyl}-1H-quinolin-2-on

121 mg (0.413 mmol) 7-Benzyloxy-5-oxiranyl-1H-quinolin-2-on, 125 mg (0.570 mmol) 1-(3-Amino-3-methyl-butyl)-1,3-dihydro-benzoimidazol-2-on und 0.4 mL Isopropanol werden zusammengegeben und 30 Minuten bei 135°C mit Mikrowellen bestrahlt. Die Reaktionsmischung wird mit Ethylacetat und 0.5 molarer Weinsäure versetzt, wobei ein Feststoff ausfällt. Der Feststoff und die wässrige Phase werden abgetrennt und es wird Wasser, Dichlormethan und etwas Methanol zugegeben. Die wässrige Phase wird mit Dichlormethan extrahiert und die vereinigten Dichlormethan-Phasen werden getrocknet und vom Lösungsmittel befreit. Der Rückstand wird mit Salzsäure in Ethylacetat versetzt, das Lösungsmittel abdestilliert und der Rückstand in Ethylacetat verrührt. Weißer Feststoff. Ausbeute: 87 mg (38%, Hydrochlorid); Massenspektroskopie: [M+H]⁺ = 513.

### b) 5-{2-[1,1-Dimethyl-3-(2-oxo-2,3-dihydro-benzoimidazol-1-yl)-propylamino]-1-hydroxyethyl}-7-hydroxy-1H-quinolin-2-on

71 mg (0.129 mmol) 7-Benzyloxy-5-{2-[1,1-dimethyl-3-(2-oxo-2,3-dihydro-benzoimidazol-1-yl)-propylamino]-1-hydroxy-ethyl}-1H-quinolin-2-on Hydrochlorid werden in Methanol gelöst und mit Palladium auf Kohle als Katalysator bei Normaldruck hydriert. Der Katalysator wird durch Filtration über Celite abgetrennt und das Filtrat vom Lösungsmittel befreit. Verrühren des Rückstands mit Ethylacetat liefert das Produkt in Form eines Feststoffs. Ausbeute: 31 mg (52%, Hydrochlorid); Massenspektroskopie: [M+H]⁺ = 423.

### Beispiel 12: 6-Hydroxy-8-{1-hydroxy-2-[4-(4-methoxy-2-oxo-2,3-dihydro-benzoimidazol-1-yl)-1,1-dimethyl-butylamino]-ethyl}-4H-benzo[1,4]oxazin-3-on

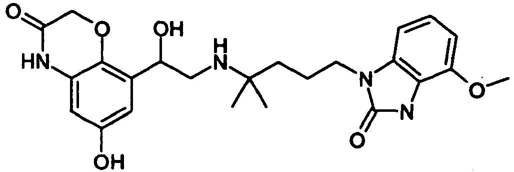

### a) 6-Benzyloxy-8-(1-hydroxy-2-[4-(4-methoxy-2-oxo-2,3-dihydro-benzoimidazol-1-yl)-1,1-dimethyl-butylamino]-ethyl}-4H-benzo[1,4]oxazin-3-on

200 mg (0.667 mmol) 1-(3-Amino-3-methyl-butyl)-4-methoxy-1,3-dihydro-benzoimidazol-2-on Hydrochlorid und 120 µL (0.733 mmol) Triethylamin in 5 mL THF werden 30 Minuten gerührt und dann mit 200 mg (0.666 mmol) 6-Benzyloxy-8-(2-ethoxy-2-hydroxy-acetyl)-4H-benzo[1,4]oxazin-3-on versetzt. Nach 2 Stunden wird die Reaktionsmischung auf 10°C gekühlt und es werden 60 mg (2.76 mmol) Lithiumborhydrid zugegeben. Es wird eine Stunde bei Raumtemperatur gerührt, anschließend auf 10°C gekühlt und mit 15 mL Wasser versetzt. Die organische Phase wird mit Dichlormethan extrahiert und die vereinigten organischen Extrakte werden getrocknet und vom Lösungsmittel befreit. Das zurückbleibende Öl wird in Ethylacetat gelöst und mit Salzsäure in Ethylacetat auf pH 2 eingestellt. Das Lösungsmittel wird abdestilliert und der Rückstand mit Dichlormethan/Diethylether verrührt. Ausbeute: 130 mg (35%, Hydrochlorid); Massenspektroskopie: [M+H]⁺ = 561.

### b) 6-Hydroxy-8-{1-hydrox-2-[4-(4-methoxy-2-oxo-2,3-dihydro-benzoimidazol-1-yl)-1,1-dimethyl-butylamino]-ethyl}-4H-benzo[1,4]oxazin-3-on

130 mg (0.213 mmol) 6-Benzyloxy-8-{1-hydroxy-2-[4-(4-methoxy-2-oxo-2,3-dihydro-benzoimidazol-1-yl)-1,1-dimethyl-butylamino]-ethyl}-4H-benzo[1,4]oxazin-3-on hydrochlorid werden in Methanol gelöst und mit Palladium auf Kohle als Katalysator bei Normaldruck hydriert. Der Katalysator wird über Celite abfiltriert, das Filtrat vom Lösungsmittel befreit und der Rückstand mit Ethylacetat verrührt. Feststoff. Ausbeute: 50 mg (45%, Hydrochlorid); Massenspektroskopie: [M+H]⁺ = 471.

### Beispiel 13: 6-Hydroxy-8-{1-hydroxy-2-[4-(5-methoxy-3-methyl-2-oxo-2,3-dihydro-benzoimidazol-1-yl)-1,1-dimethyl-butylamino]-ethyl}-4H-benzo[1,4]oxazin-3-on

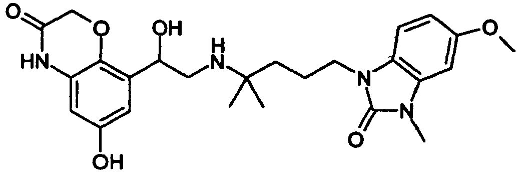

Hergestellt aus 1-(3-Amino-3-methyl-butyl)-5-methoxy-3-methyl-1,3-dihydro-benzoimidazol-2-on und 6-Benzyloxy-8-(2-ethoxy-2-hydroxy-acetyl)-4H-benzo[1,4]oxazin-3-on in Analogie zu dem für Beispiel 13 beschriebenen Verfahren. Massenspektroskopie: [M+H]⁺ = 485.

### Beispiel 14: 8-{2-[4-(6-Fluor-3-methyl-2-oxo-2,3-dihydro-benzoimidazol-1-yl)-1,1-dimethyl-butylamino]-1-hydroxy-ethyl}-6-hydroxy-4H-benzo[1,4]oxazin-3-on

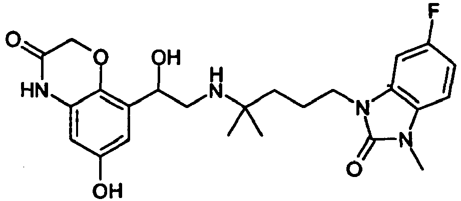

### a) 6-Benzyloxy-8-{2-[4-(6-fluor-3-methyl-2-oxo-2,3-dihydro-benzoimidazol-1-yl)-1,1-dimethyl-butylamino]-1-hydroxy-ethyl}-4H-benzo[1,4]oxazin-3-on

200 mg (0.754 mmol) 3-(4-Amino-4-methyl-pentyl)-5-fluor-1-methyl-1,3-dihydro-benzoimidazol-2-on Hydrochlorid und 237 mg (0.663 mmol) 6-Benzyloxy-8-(2-ethoxy-2-hydroxy-acetyl)-4H-benzo[1,4]oxazin-3-on werden in Analogie zu der Vorschrift für Beispiel 13a umgesetzt Die abschließende Reinigung erfolgt mittels Chromatographie an einer Kieselgelsäule. Ausbeute: 164 mg (44%); Massenspektroskopie: [M+H]⁺ = 563.

### b) 8-{2-[4-(6-Fluor-3-methyl-2-oxo-2,3-dihydro-benzoimidazol-1-yl)-1,1-dimethyl-butylamino]-1-hydroxy-ethyl}-6-hydroxy-4H-benzo[1,4]oxazin-3-on

164 mg (0.274 mmol) 6-Benzyloxy-8-{2-[4-(6-Fluor-3-methyl-2-oxo-2,3-dihydro-benzoimidazol-1-yl)-1,1-dimethyl-butylamino]-1-hydroxy-ethyl}-4H-benzo[1,4]oxazin-3-on werden analog zur Vorschrift für Beispiel 13b debenzyliert. Zur Reinigung wird das Rohprodukt mit Ethylacetat verrührt. Massenspektroskopie: [M+H]⁺ = 473.

### Beispiel 15: 8-{2-[4-(7-Fluor-3-methyl 2-oxo-2,3-dihydro-benzoimidazol-1-yl)-1,1-dimethyl-butylamino]-1-hydroxy-ethyl}-6-hydroxy-4H-benzo[1,4]oxazin-3-on

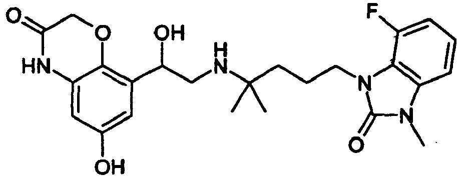

### a) 6-Benzyloxy-8-{2-[4-(7-fluor-3-methyl-2-oxo-2,3-dihydro-benzoimidazol-1-yl)-1,1-dimethyl-butylamino]-1-hydroxy-ethyl}-4H-benzo[1,4]oxazin-3-on

200 mg (0.663 mmol) 3-(4-Amino-4-methyl-pentyl)-4-fluor-1-methyl-1,3-dihydro-benzoimidazol-2-on Hydrochlorid und 237 mg (0.663 mmol) 6-Benzyloxy-8-(2-ethoxy-2-hydroxy-acetyl)-4H-benzo[1,4]oxazin-3-on werden analog der Vorschrift zur Herstellung von Beispiel 13a umgesetzt. Die abschließende Reinigung des Produkts erfolgt mittels Chromatographie an einer Kieselgelsäule. Ausbeute: 68 mg (17%); Massenspektroskopie: [M+H]⁺ = 563.

### b) 8-{2-[4-(7-Fluor-3-methyl-2-oxo-2,3-dihydro-benzoimidazol-1-yl)-1,1-dimethyl-butylamino]-1-hydroxy-ethyl}-6-hydroxy-4H-benzo[1,4]oxazin-3-on

68 mg (0.121 mmol) 6-Benzyloxy-8-{2-[4-(7-fluor-3-methyl-2-oxo-2,3-dihydro-benzoimidazol-1-yl)-1,1-dimethyl-butylamino]-1-hydroxy-ethyl}-4H-benzo[1,4]oxazin-3-on werden nach dem für Beispiel 13b beschriebenen Verfahren debenzyliert. Zur Reinigung wird das Rohprodukt in Ethylacetat verrührt. Ausbeute: 60 mg; Massenspektroskopie: [M+H]⁺ = 474.

Geeignete Anwendungsformen zur Applikation der Verbindungen der Formel 1 sind beispielsweise Tabletten, Kapseln, Zäpfchen, Lösungen, Pulver etc. Der Anteil der pharmazeutisch wirksamen Verbindung(en) sollte jeweils im Bereich von 0,05 bis 90 Gew.-%, bevorzugt 0,1 bis 50 Gew.-% der Gesamtzusammensetzung liegen. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können. Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Lösungen werden in üblicher Weise, z.B. unter Zusatz von Isotonantien, Konservierungsmitteln, wie p-Hydroxybenzoaten, oder Stabilisatoren, wie Alkalisalzen der Ethylendiamintetraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösungsmittel als Lösevermittler bzw. Hilfslösungsmittel eingesetzt werden können, hergestellt und in Injektionsflaschen oder Ampullen oder Infusionsflaschen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt. Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösungsmittel, wie Paraffine (z.B. Erdölfraktionen), Öle pflanzlichen Ursprungs (z.B. Erdnuß- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide), synthetische Gesteinsmehle (z.B. hochdisperse Kieselsäure und Silikate), Zucker (z.B. Rohr-, Milch- und Traubenzucker), Emulgiermittel (z.B. Lignin, Sufitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserem oder Farbstoffen versetzt werden.

Bei der erfindungsgemäßen Applikation der Verbindungen der Formel 1 zur Therapie von Atemwegserkrankungen werden besonders bevorzugt inhalativ applizierbare Darreichungsformen bzw. pharmazeutische Formulierungen eingesetzt. Als inhalierbare Darreichungsformen kommen Inhalationspulver, treibgashaltige Dosieraerosole oder treibgasfreie Inhalationslösungen in Betracht. Im Rahmen der vorliegenden Erfindung sind von dem Begriff treibgasfreie Inhalationslösungen auch Konzentrate oder sterile, gebrauchsfertige Inhalationslösungen umfaßt. Die im Rahmen der vorliegenden Erfindung einsetzbaren Darreichungsformen werden im nachfolgenden Teil der Beschreibung detailliert beschrieben.

Erindungsgemäß einsetzbare Inhalationspulver können 1 entweder allein oder im Gemisch mit geeigneten physiologisch unbedenkliche Hilfsstoffen enthalten. Sind die Wirkstoffe 1 im Gemisch mit physiologisch unbedenklichen Hilfsstoffen enthalten, können zur Darstellung dieser erfindungsgemäßen Inhalationspulver die folgenden physiologisch unbedenklichen Hilfsstoffe zur Anwendung gelangen: Monosaccharide (z.B. Glucose oder Arabinose), Disaccharide (z.B. Lactose, Saccharose, Maltose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist Als besonders bevorzugt im Sinne der Erfindung gelangt Lactose, höchst bevorzugt Lactosemonohydrat als Hilfsstoff zur Anwendung.
Die Hilfsstoffe weisen im Rahmen der erfindungsgemäßen Inhalationspulver eine maximale mittlere Teilchengröße von bis zu 250µm, bevorzugt zwischen 10 und 150µm, besonders bevorzugt zwischen 15 und 80µm auf. Gegebenenfalls kann es sinnvoll erscheinen, den vorstehend genannten Hilfsstoffen feinere Hilfsstofffraktionen mit einer mittleren Teilchengröße von 1 bis 9µm beizumischen. Letztgenannte feinere Hilfsstoffe sind ebenfalls ausgewählt aus der vorstehend genannten Gruppe an einsetzbaren Hilfsstoffen. Schließlich wird zur Herstellung der erfindungsgemäßen Inhalationspulver mikronisierter Wirkstoff 1, vorzugsweise mit einer mittleren Teilchengröße von 0,5 bis 10µm, besonders bevorzugt von 1 bis 5µm, der Hilfsstoffmischung beigemischt. Verfahren zur Herstellung der erfindungsgemäßen Inhalationspulver durch Mahlen und Mikronisieren sowie durch abschließendes Mischen der Bestandteile sind aus dem Stand der Technik bekannt.

Die erfindungsgemäßen Inhalationspulver können mittels aus dem Stand der Technik bekannten Inhalatoren appliziert werden.

Erfindungsgemäße treibgashaltige Inhalationsaerosole können 1 im Treibgas gelöst oder in dispergierter Form enthalten. Die zur Herstellung der Inhalationsaerosole einsetzbaren Treibgase sind aus dem Stand der Technik bekannt. Geeignete Treibgase sind ausgewählt aus der Gruppe bestehend aus Kohlenwasserstoffen wie n-Propan, n-Butan oder Isobutan und Halogenkohlenwasserstoffen wie fluorierten Derivaten des Methans, Ethans, Propans, Butans, Cyclopropans oder Cyclobutans. Die vorstehend genannten Treibgase können dabei allein oder in Mischungen derselben zur Verwendung kommen. Besonders bevorzugte Treibgase sind halogenierte Alkanderivate ausgewählt aus TG134a und TG227 und Mischungen derselben.

Die treibgashaltigen Inhalationsaerosole können ferner weitere Bestandteile wie Kosolventien, Stabilisatoren, oberflächenaktive Mittel (surfactants), Antioxidantien, Schmiermittel sowie Mittel zur Einstellung des pH-Werts enthalten. All diese Bestandteile sind im Stand der Technik bekannt.

Die vorstehend genannten treibgashaltigen Inhalationsaerosole können mittels im Stand der Technik bekannten Inhalatoren (MDIs = metered dose inhalers) appliziert werden.

Ferner kann die Applikation der erfindungsgemäßen Wirkstoffe 1 in Form von treibgasfreien Inhalationslösungen und Inhalationssuspensionen. Als Lösungsmittel kommen hierzu wässrige oder alkoholische, bevorzugt ethanolische Lösungen in Betracht. Das Lösungsmittel kann ausschließlich Wasser sein oder es ist ein Gemisch aus Wasser und Ethanol. Der relative Anteil an Ethanol gegenüber Wasser ist nicht begrenzt, bevorzugt liegt die maximale Grenze jedoch bei bis 70 Volumenprozent, insbesondere bei bis zu 60 Volumenprozent und besonders bevorzugt bei bis zu 30 Volumenprozent. Die restlichen Volumenprozente werden von Wasser aufgefüllt. Die 1 enthaltenden Lösungen oder Suspensionen werden mit geeigneten Säuren auf einen pH-Wert von 2 bis 7, bevorzugt von 2 bis 5 eingestellt. Zur Einstellung dieses pH-Werts können Säuren ausgewählt aus anorganischen oder organischen Säuren Verwendung finden. Beispiele für besonders geeignete anorganische Säuren sind Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure und andere. Bevorzugte anorganische Säuren sind Salzsäure und Schwefelsäure. Es können auch die Säuren verwendet werden, die bereits mit einem der Wirkstoffe ein Säureadditionssalz bilden. Unter den organischen Säuren sind Ascorbinsäure, Fumarsäure und Zitronensäure bevorzugt. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe, Antioxidantien oder Komplexbildner besitzen, wie beispielsweise Zitronensäure oder Ascorbinsäure. Erfindungsgemäß besonders bevorzugt wird Salzsäure zur Einstellung des pH-Werts verwendet.
In diesen Formulierungen kann gegebenenfalls auf den Zusatz von Editinsäure (EDTA) oder einem der bekannten Salze davon, Natriumedetat, als Stabilisator oder Komplexbildner verzichtet werden. Andere Ausführungsformen beinhalten diese Verbindung(en). In einer solchen bevorzugten Ausführungsform liegt der Gehalt bezogen auf Natriumedetat unter 100 mg / 100 ml, bevorzugt unter 50 mg/ 100ml, besonders bevorzugt unter 20 mg/ 100ml. Generell sind solche Inhalationslösungen bevorzugt, in denen der Gehalt an Natriumedetat bei 0 bis 10mg/100ml liegt.

Den treibgasfreien Inhalationslösungen können Co-Solventien und/oder weitere Hilfsstoffe zugesetzt werden. Bevorzugte Co-Solventien ind solche, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester. Unter Hilfs- und Zusatzstoffen wird in diesem Zusammenhang jeder pharmakologisch verträgliche Stoff verstanden, der kein Wirkstoff ist, aber zusammen mit dem (den) Wirkstoff(en) in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. oberflächenaktive Stoffe, wie z.B. Sojalecithin, Ölsäure, Sorbitanester, wie Polysorbate, Polyvinylpyrrolidon sonstige Stabilisatoren, Komplexbildner, Antioxidantien und/oder Konservierungsstoffe, die die Verwendungsdauer der fertigen Arzneimittelformulierung gewährleisten oder verlängern, Geschmackstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid als Isotonantien.

Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine.

Konservierungsstoffe können eingesetzt werden, um die Formulierung vor Kontamination mit Keimen zu schützen. Als Konservierungsstoffe eignen sich die dem Stand der Technik bekannten, insbesondere Cetylpyridiniumchlorid, Benzalkoniumchlorid oder Benzoesäure bzw. Benzoate wie Natriumbenzoat in der aus dem Stand der Technik bekannten Konzentration. Die vorstehend genannten Konservierungsstoffe sind vorzugsweise in Konzentrationen von bis zu 50mg/100ml, besonders bevorzugt zwischen 5 und 20 mg/100ml enthalten.

Bevorzugte Formulierungen enthalten außer dem Lösungsmittel Wasser und dem Wirkstoff 1 nur noch Benzalkoniumchlorid und Natriumedetat.

In einer anderen bevorzugten Ausführungsform wird auf Natriumedetat verzichtet.

Die Dosierung der erfindungsgemäßen Verbindungen ist naturgemäß stark von der Applikationsart und der zu therapierenden Erkrankung abhängig. Bei inhalativer Applikation zeichnen sich die Verbindungen der Formel 1 bereits bei Dosen im µg-Bereich durch eine hohe Wirksamkeit aus. Auch oberhalb des µg-Bereichs, lassen sich die Verbindungen der Formel 1 sinnvoll einsetzen. Die Dosierung kann dann beispielsweise auch im Milligrammbereich liegen.

Ein weiterer Aspekt der vorliegenden Erfindung betrifft die vorstehend genannten pharmazeutischen Formulierungen, gekennzeichnet durch einen Gehalt einer Verbindung der Formel 1, als solche, besonders bevorzugt die vorstehend genannten inhalativ applizierbaren pharmazeutischen Formulierungen.

Die nachfolgenden Formulierungsbeipiele illustrieren die vorliegende Erfindung ohne sie jedoch in ihrem Umfang zu beschränken:

| A) | Ampullenlösung | |
|---|---|---|
| | Wirkstoff der Formel 1 | 25 mg |
| | Natriumchlorid | 50 mg |
| | Aqua pro inj. | 5 ml |

Der Wirkstoff wird bei Eigen-pH oder gegebenenfalls bei pH 5,5 bis 6,5 in Wasser gelöst und mit Natriumchlorid als Isotonans versetzt. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

| B) | Dosieraerosol (Suspension) | |
|---|---|---|
| | Wirkstoff der Formel 1 | 0. 3 Gew.% |
| | Sorbitantrioleat | 0.6 Gew. % |
| | HFA134A:HFA227 2:1 | 99.1 Gew.% |

Die Suspension wird in einen üblichen Aerosolbehälter mit Dosierventil gefüllt. Pro Betätigung werden vorzugsweise 50 µl Suspension abgegeben. Der Wirkstoff kann gewünschtenfalls auch höher dosiert werden

| C) | Dosieraerosol (Lösung) | |
|---|---|---|
| | Wirkstoff der Formel 1 | 0. 3 Gew.% |
| | Ethanol abs. | 20 Gew.% |
| | wässrige HCl 0.01 mol/l | 2.0 Gew.% |
| | HFA134A | 77.7 Gew.% |

Die Herstellung der Lösung erfolgt in üblicher Art und Weise durch Mischen der einzelnen Bestandteile.

| D) | Inhalationspulver | |
|---|---|---|
| | Wirkstoff der Formel 1 | 80 µg |
| | Lactose Monohydrat | ad 10 mg |

Die Herstellung des Inhalationspulvers erfolgt in üblicher Art und Weise durch Mischen der einzelnen Bestandteile.

## Patentansprüche

1. Verbindungen der Formel 1 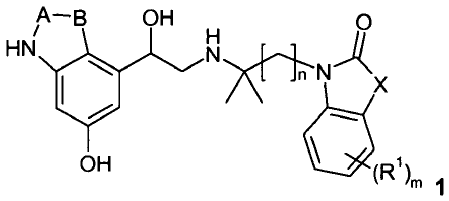 worin
n 1, 2, 3 oder 4;
m 1, 2 oder 3;
X CH₂, CO, NR², S oder O;
A CO;
B eine zweibindige Gruppe ausgewählt aus der Gruppe bestehend aus O, S, CH₂, CR³R⁴-O, CR³R⁴-S, NR⁵, CR³R⁴-NR⁵, CH=CH oder CH₂-CH₂;
R¹ H, C₁₋₆-Alkyl, C₂₋₆-Alkenyl, C₂₋₆-Alkinyl, C₃₋₆-Cycloalkyl, C₁₋₆-Haloalkyl, O-C₁₋₆-Haloalkyl, Halogen, OH, CN, NO₂, O-C₁₋₆-Alkyl, COOH oder COO-C₁₋₄-Alkyl;
R² H, C₁₋₆-Alkyl, C₁₋₄-Alkylen-C₆-C₁₀-aryl oder C₁₋₄-Alkylen-C₃₋₆-cycloalkyl;
R³ H oder C₁₋₆-Alkyl;
R⁴ H oder C₁₋₆-Alkyl;
R⁵ H oder C₁₋₆-Alkyl;
bedeuten, gegebenenfalls in Form der einzelnen Enantiomere, Mischungen der einzelnen Enantiomere oder Racemate, gegebenenfalls in Form ihrer Säureadditionssalze mit pharmakologisch unbedenklichen Säuren sowie gegebenenfalls in Form der Solvate und/oder Hydrate.

2. Verbindungen der Formel 1, nach Anspruch 1, worin
n 1, 2, 3 oder 4;
m 1, 2 oder 3;
X CH₂, CO, NR², S oder O;
A CO;
B eine zweibindige Gruppe ausgewählt aus der Gruppe bestehend aus O, S, CH₂, CR³R⁴-O, CR³R⁴-S, NR⁵, CR³R⁴-NR⁵, CH=CH oder CH₂-CH₂;
R¹ H, C₁₋₆-Alkyl, C₁₋₆-Haloalkyl, C₃₋₆-Cycloalkyl, Halogen, OH,CN, NO₂, O-C₁₋₆-Alkyl, COOH oder COO-C₁₋₄-Alkyl;
R² H, C₁₋₄-Alkyl, C₁₋₂-Alkylen-C₃₋₆-cycloalkyl, Phenylethyl oder Benzyl;
R³ H oder C₁₋₆-Alkyl;
R⁴ H oder C₁₋₆-Alkyl;
R⁵ H oder C₁₋₆-Alkyl;
bedeuten, gegebenenfalls in Form der einzelnen Enantiomere, Mischungen der einzelnen Enantiomere oder Racemate, gegebenenfalls in Form ihrer Säureadditionssalze mit pharmakologisch unbedenklichen Säuren sowie gegebenenfalls in Form der Solvate und/oder Hydrate.

3. Verbindungen der Formel 1, nach einem der Ansprüche 1 oder 2, worin
n 2 oder 3;
m 1, 2 oder 3;
X CH₂, CO, NR², S oder O;
A CO;
B eine zweibindige Gruppe ausgewählt aus der Gruppe bestehend aus O, S, CH₂, CR³R⁴-O, CR³R⁴-S, NR⁵, CR³R⁴-NR⁵, CH=CH oder CH₂-CH₂;
R¹ H, Methyl, Ethyl, Propyl, CF₃, CH₂F, CH₂CF₃, Fluor, Chlor, Brom, OH, Methoxy, Ethoxy, COOH oder COOMe;
R² H, Methyl, Ethyl oder Propyl;
R³ H, Methyl, Ethyl oder Propyl;
R⁴ H, Methyl, Ethyl oder Propyl;
R⁵ H, Methyl, Ethyl oder Propyl;
bedeuten, gegebenenfalls in Form der einzelnen Enantiomere, Mischungen der einzelnen Enantiomere oder Racemate, gegebenenfalls in Form ihrer Säureadditionssalze mit pharmakologisch unbedenklichen Säuren sowie gegebenenfalls in Form der Solvate und/oder Hydrate.

4. Verbindungen der Formel 1, nach einem der Ansprüche 1 bis 3, worin
n 2 oder 3;
m 1, 2 oder 3;
X CH₂, CO, NR², S oder O;
A CO;
B eine zweibindige Gruppe ausgewählt aus der Gruppe bestehend aus CH₂-O, CH=CH oder CH₂-CH₂;
R¹ H, Methyl, Ethyl, Propyl, CF₃, CH₂F, CH₂CF₃, Fluor, Chlor, Brom, OH, Methoxy, Ethoxy, COOH oder COOMe;
R² H, Methyl, Ethyl oder Propyl;
bedeuten, gegebenenfalls in Form der einzelnen Enantiomere, Mischungen der einzelnen Enantiomere oder Racemate, gegebenenfalls in Form ihrer Säureadditionssalze mit pharmakologisch unbedenklichen Säuren sowie gegebenenfalls in Form der Solvate und/oder Hydrate.

5. Verbindungen der Formel 1, nach einem der Ansprüche 1 bis 4, worin
n 2 oder 3;
m 1 oder 2;
X CH₂, CO, NR², S oder O;
A CO;
B eine zweibindige Gruppe ausgewählt aus der Gruppe bestehend aus CH₂-O, CH=CH oder CH₂-CH₂;
R¹ H, Methyl, Ethyl, Propyl, CF₃, CH₂F oder CH₂CF₃;
R² H, Methyl, Ethyl oder Propyl;
bedeuten, gegebenenfalls in Form der einzelnen Enantiomere, Mischungen der einzelnen Enantiomere oder Racemate, gegebenenfalls in Form ihrer Säureadditionssalze mit pharmakologisch unbedenklichen Säuren sowie gegebenenfalls in Form der Solvate und/oder Hydrate.

6. Verbindungen der Formel 1, nach einem der Ansprüche 1 bis 5, worin
n 2 oder 3;
m 1;
X CH₂, CO, NR², S oder O;
A CO;
B eine zweibindige Gruppe ausgewählt aus der Gruppe bestehend aus CH₂-O, CH=CH oder CH₂-CH₂;
R¹ H, Methyl oder CF₃;
R² H oder Methyl;
bedeuten, gegebenenfalls in Form der einzelnen Enantiomere, Mischungen der einzelnen Enantiomere oder Racemate, gegebenenfalls in Form ihrer Säureadditionssalze mit pharmakologisch unbedenklichen Säuren sowie gegebenenfalls in Form der Solvate und/oder Hydrate.

7. Verbindungen der Formel 1, nach einem der Ansprüche 1 bis 6,
X NR², O; worin R² die in den Ansprüchen 1 bis 7 genannte Bedeutung hat;
bedeutet, gegebenenfalls in Form der einzelnen Enantiomere, Mischungen der einzelnen Enantiomere oder Racemate, gegebenenfalls in Form ihrer Säureadditionssalze mit pharmakologisch unbedenklichen Säuren sowie gegebenenfalls in Form der Solvate und/oder Hydrate.

8. Verbindungen der Formel 1, nach einem der Ansprüche 1 bis 7, worin
n 2 oder 3;
m 1;
X NR², O;
A CO;
B eine zweibindige Gruppe ausgewählt aus der Gruppe bestehend aus CH₂-O oder CH=CH;
R¹ H, Methyl oder CF_{3;}
R² H oder Methyl;
bedeuten, gegebenenfalls in Form der einzelnen Enantiomere, Mischungen der einzelnen Enantiomere oder Racemate, gegebenenfalls in Form ihrer Säureadditionssalze mit pharmakologisch unbedenklichen Säuren sowie gegebenenfalls in Form der Solvate und/oder Hydrate.

9. Verbindungen der Formel 1, nach einem der Ansprüche 1 bis 8, worin
n 2;
m 1;
X NH;
A CO;
B eine zweibindige Gruppe CH₂-O;
R¹ H, Methyl oder CF_{3;}
bedeutet, gegebenenfalls in Form der einzelnen Enantiomere, Mischungen der einzelnen Enantiomere oder Racemate, gegebenenfalls in Form ihrer Säureadditionssalze mit pharmakologisch unbedenklichen Säuren sowie gegebenenfalls in Form der Solvate und/oder Hydrate.

10. Verbindungen der Formel 1 nach einem der Ansprüche 1 bis 9, **dadurch gekennzeichnet, dass** sie in Form eines ihrer Säureadditionssalze mit pharmakologisch unbedenklichen Säuren vorliegen, welches ausgewählt ist aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat.

11. Verbindungen der Formel 1 nach einem der Ansprüche 1 bis 10, **dadurch gekennzeichnet, dass** sie in Form der R-Enantiomere der Formel R-1 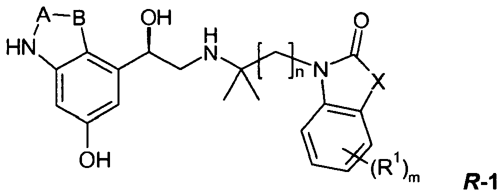 vorliegen.

12. Verbindungen der Formel 1 gemäß einem der Ansprüche 1 bis 11 als Arzneimittel.

13. Verwendung der Verbindungen der Formel 1 gemäß einem der Ansprüche 1 bis 12 zur Herstellung eines Arzneimitels zur Behandlung von Atemwegserkrankungen.

14. Pharmazeutische Zusammensetzung, **gekennzeichnet durch** einen Gehalt einer Verbindung der Formel 1 gemäß einem der Ansprüche 1 bis 12.

## Claims

1. Compounds of formula 1 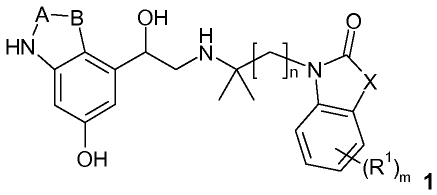 wherein
n denotes 1, 2, 3 or 4;
m denotes 1, 2 or 3;
X denotes CH₂, CO, NR², S or O;
A denotes CO;
B denotes a double-bonded group selected from among O, S, CH₂, CR³R⁴-O, CR³R⁴-S, NR⁵, CR³R⁴-NR⁵, CH=CH or CH₂-CH₂;
R¹ denotes H, C₁₋₆-alkyl, C₂₋₆-alkenyl, C₂₋₆-alkynyl, C₃₋₆-cycloalkyl, C₁₋₆-haloalkyl, O-C₁₋₆-haloalkyl, halogen, OH, CN, NO₂, O-C₁₋₆-alkyl, COOH or COO-C₁₋₄-alkyl;
R² denotes H, C₁₋₆-alkyl, C₁₋₄-alkylene-C₆-C₁₀-aryl or C₁₋₄-alkylene-C₃₋₆-cycloalkyl;
R³ denotes H or C₁₋₆-alkyl;
R⁴ denotes H or C₁₋₆-alkyl;
R⁵ denotes H or C₁₋₆-alkyl;
optionally in the form of the individual enantiomers, mixtures of the individual enantiomers or racemates, optionally in the form of the acid addition salts thereof with pharmacologically acceptable acids and optionally in the form of the solvates and/or hydrates thereof.

2. Compounds of formula 1, according to claim 1, wherein
n denotes 1, 2, 3 or 4;
m denotes 1, 2 or 3;
X denotes CH₂, CO, NR², S or O;
A denotes CO;
B denotes a double-bonded group selected from among O, S, CH₂, CR³R⁴-O, CR³R⁴-S, NR⁵, CR³R⁴-NR⁵, CH=CH or CH₂-CH₂;
R¹ denotes H, C₁₋₆-alkyl, C₁₋₆-haloalkyl, C₃₋₆-cycloalkyl, halogen, OH, CN, NO₂, O-C₁₋₆-alkyl, COOH or COO-C₁₋₄-alkyl;
R² denotes H, C₁₋₄-alkyl, C₁₋₂-alkylene-C₃₋₆-cycloalkyl, phenylethyl or benzyl;
R³ denotes H or C₁₋₆-alkyl;
R⁴ denotes H or C₁₋₆-alkyl;
R⁵ denotes H or C₁₋₆-alkyl;
optionally in the form of the individual enantiomers, mixtures of the individual enantiomers or racemates, optionally in the form of the acid addition salts thereof with pharmacologically acceptable acids and optionally in the form of the solvates and/or hydrates thereof.

3. Compounds of formula 1, according to one of claims 1 or 2, wherein
n denotes 2 or 3;
m denotes 1, 2 or 3;
X denotes CH₂, CO, NR², S or O;
A denotes CO;
B denotes a double-bonded group selected from among O, S, CH₂, CR³R⁴-O, CR³R⁴-S, NR⁵, CR³R⁴-NR⁵, CH=CH or CH₂-CH₂;
R¹ denotes H, methyl, ethyl, propyl, CF₃, CH₂F, CH₂CF₃, fluorine, chlorine, bromine, OH, methoxy, ethoxy, COOH or COOMe;
R² denotes H, methyl, ethyl or propyl;
R³ denotes H, methyl, ethyl or propyl;
R⁴ denotes H, methyl, ethyl or propyl;
R⁵ denotes H, methyl, ethyl or propyl;
optionally in the form of the individual enantiomers, mixtures of the individual enantiomers or racemates, optionally in the form of the acid addition salts thereof with pharmacologically acceptable acids and optionally in the form of the solvates and/or hydrates thereof.

4. Compounds of formula 1, according to one of claims 1 to 3, wherein
n denotes 2 or 3;
m denotes 1, 2 or 3;
X denotes CH₂, CO, NR², S or O;
A denotes CO;
B denotes a double-bonded group selected from among CH₂-O, CH=CH or CH₂-CH₂;
R¹ denotes H, methyl, ethyl, propyl, CF₃, CH₂F, CH₂CF₃, fluorine, chlorine, bromine, OH, methoxy, ethoxy, COOH or COOMe;
R² denotes H, methyl, ethyl or propyl;
optionally in the form of the individual enantiomers, mixtures of the individual enantiomers or racemates, optionally in the form of the acid addition salts thereof with pharmacologically acceptable acids and optionally in the form of the solvates and/or hydrates thereof.

5. Compounds of formula 1, according to one of claims 1 to 4, wherein
n denotes 2 or 3;
m denotes 1 or 2;
X denotes CH₂, CO, NR², S or O;
A denotes CO;
B denotes a double-bonded group selected from among CH₂-O, CH=CH or CH₂-CH₂;
R¹ denotes H, methyl, ethyl, propyl, CF₃, CH₂F or CH₂CF₃;
R² denotes H, methyl, ethyl or propyl;
optionally in the form of the individual enantiomers, mixtures of the individual enantiomers or racemates, optionally in the form of the acid addition salts thereof with pharmacologically acceptable acids and optionally in the form of the solvates and/or hydrates thereof.

6. Compounds of formula 1, according to one of claims 1 to 5, wherein
n denotes 2 or 3;
m denotes 1 ;
X denotes CH₂, CO, NR², S or O;
A denotes CO;
B denotes a double-bonded group selected from among CH₂-O, CH=CH or CH₂-CH₂;
R¹ denotes H, methyl or CF_{3;}
R² denotes H or methyl;
optionally in the form of the individual enantiomers, mixtures of the individual enantiomers or racemates, optionally in the form of the acid addition salts thereof with pharmacologically acceptable acids and optionally in the form of the solvates and/or hydrates thereof.

7. Compounds of formula 1, according to one of claims 1 to 6, wherein
X denotes NR², O; wherein R² has the meaning given in claims 1 to 7;
optionally in the form of the individual enantiomers, mixtures of the individual enantiomers or racemates, optionally in the form of the acid addition salts thereof with pharmacologically acceptable acids and optionally in the form of the solvates and/or hydrates thereof.

8. Compounds of formula 1, according to one of claims 1 to 7, wherein
n denotes 2 or 3;
m denotes 1 ;
X denotes NR², O;
A denotes CO;
B denotes a double-bonded group selected from among CH₂-O or CH=CH;
R¹ denotes H, methyl or CF_{3;}
R² denotes H or methyl;
optionally in the form of the individual enantiomers, mixtures of the individual enantiomers or racemates, optionally in the form of the acid addition salts thereof with pharmacologically acceptable acids and optionally in the form of the solvates and/or hydrates thereof.

9. Compounds of formula 1, according to one of claims 1 to 8, wherein
n denotes 2;
m denotes 1;
X denotes NH;
A denotes CO;
B denotes a double-bonded group CH₂-O;
R¹ denotes H, methyl or CF_{3;}
optionally in the form of the individual enantiomers, mixtures of the individual enantiomers or racemates, optionally in the form of the acid addition salts thereof with pharmacologically acceptable acids and optionally in the form of the solvates and/or hydrates thereof.

10. Compounds of formula 1 according to one of claims 1 to 9, **characterised in that** they are in the form of one of the acid addition salts thereof with pharmacologically acceptable acids which is selected from among the hydrochloride, hydrobromide, hydriodide, hydrosulphate, hydrophosphate, hydromethanesulphonate, hydronitrate, hydromaleate, hydroacetate, hydrobenzoate, hydrocitrate, hydrofumarate, hydrotartrate, hydroxalate, hydrosuccinate, hydrobenzoate and hydro-p-toluenesulphonate.

11. Compounds of formula 1 according to one of claims 1 to 10, **characterised in that** they are in the form of the R-enantiomers of formula R-1

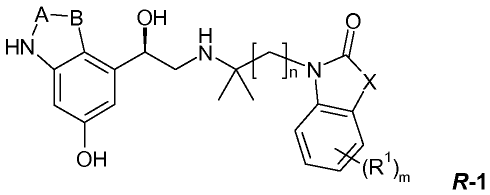

12. Compounds of formula 1 according to one of claims 1 to 11 as medicaments.

13. Use of the compounds of formula 1 according to one of claims 1 to 12 for preparing a medicament for the treatment of respiratory complaints.

14. Pharmaceutical composition, **characterised in that** it contains a compound of formula 1 according to one of claims 1 to 12.

## Revendications

1. Composés de formule 1 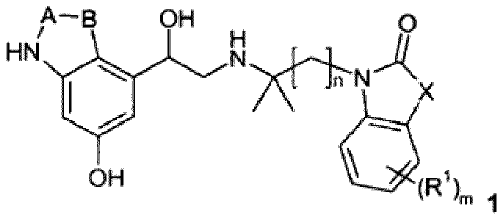 dans laquelle
n est 1, 2, 3 ou 4 ;
m est 1, 2 ou 3 ;
X est CH₂, CO, NR², S ou O ;
A est CO ;
B est un groupe à double liaison choisi dans le groupe comprenant 0, S, CH₂, CR³R⁴-O, CR³R⁴-S, NR⁵, CR³R⁴-NR⁵, CH=CH ou CH₂-CH₂ ;
R¹ est H, un groupe alkyle en C₁ à C₆, alcényle en C₂ à C₆, alcinyle en C₂ à C₆, cycloalkyle en C₃ à C₆, halogénoalkyle en C₁ à C₆, O-halogénoalkyle en C₁ à C₆, un halogène, OH, CN, NO₂, O-alkyle en C₁ à C₆, COOH ou COO-alkyle en C₁ à C₄ ;
R² est H, un groupe alkyle en C₁ à C₆, alkylène en C₁ à C₄-aryle en C₆ à C₁₀ ou alkylène en C₁ à C₄-cycloalkyle en C₃ à C₆ ;
R³ est H ou un groupe alkyle en C₁ à C₆ ;
R⁴ est H ou un groupe alkyle en C₁ à C₆ ;
R⁵ est H ou un groupe alkyle en C₁ à C₆ ;
éventuellement sous la forme des énantiomères individuels, de mélanges des énantiomères individuels ou de racémates, éventuellement sous la forme de leurs sels d'addition acide avec des acides pharmacologiquement inoffensifs et éventuellement sous la forme de solvates et/ou hydrates.

2. Composés de formule 1, selon la revendication 1, dans laquelle
n est 1, 2, 3 ou 4 ;
m est 1, 2 ou 3 ;
X est CH₂, CO, NR², S ou O ;
A est CO ;
B est un groupe à double liaison choisi dans le groupe comprenant O, S, CH₂, CR³R⁴-O, CR³R⁴-S, NR⁵, CR³R⁴-NR⁵, CH=CH ou CH₂-CH₂ ;
R¹ est H, un groupe alkyle en C₁ à C₆, halogénoalkyle en C₁ à C₆, cycloalkyle en C₃ à C₆, un halogène, OH, CN, NO₂, O-alkyle en C₁ à C₆, COOH ou COO-alkyle en C₁ à C₄ ;
R² est H, un groupe alkyle en C₁ à C₄, alkylène e n C₁ à C₂-cycloalkyle en C₃ à C₆, phényléthyle ou benzyle ;
R³ est H ou un groupe alkyle en C₁ à C₆ ;
R⁴ est H ou un groupe alkyle en C₁ à C₆ ;
R⁵ est H ou un groupe alkyle en C₁ à C₆ ;
éventuellement sous la forme des énantiomères individuels, de mélanges des énantiomères individuels ou de racémates, éventuellement sous la forme de leurs sels d'addition acide avec des acides pharmacologiquement inoffensifs et éventuellement sous la forme de solvates et/ou hydrates.

3. Composés de formule 1, selon l'une des revendications 1 ou 2, dans laquelle
n est 2 ou 3 ;
m est 1, 2 ou 3 ;
X est CH₂, CO, NR², S ou O ;
A est CO ;
B est un groupe à double liaison choisi dans le groupe comprenant 0, S, CH₂, CR³R⁴-O, CR³R⁴-S, NR⁵, CR³R⁴-NR⁵, CH=CH ou CH₂-CH₂ ;
R¹ est H, un groupe méthyle, éthyle, propyle, CF₃, CH₂F, CH₂CF₃, un atome de fluor, de chlore, de brome, OH, un groupe méthoxy, éthoxy, COOH ou COOMe ;
R² est H, un groupe méthyle, éthyle ou propyle ;
R³ est H, un groupe méthyle, éthyle ou propyle ;
R⁴ est H, un groupe méthyle, éthyle ou propyle ;
R⁵ est H, un groupe méthyle, éthyle ou propyle ;
éventuellement sous la forme des énantiomères individuels, de mélanges des énantiomères individuels ou de racémates, éventuellement sous la forme de leurs sels d'addition acide avec des acides pharmacologiquement inoffensifs et éventuellement sous la forme de solvates et/ou hydrates.

4. Composés de formule 1, selon l'une des revendications 1 à 3, dans laquelle
n est 2 ou 3 ;
m est 1, 2 ou 3 ;
X est CH₂, CO, NR², S ou O ;
A est CO ;
B est un groupe à double liaison choisi dans le groupe comprenant CH₂-O, CH=CH ou CH₂-CH₂ ;
R¹ est H, un groupe méthyle, éthyle, propyle, CF₃, CH₂F, CH₂CF₃, un atome de fluor, de chlore, de brome, OH, un groupe méthoxy, éthoxy, COOH ou COOMe ;
R² est H, un groupe méthyle, éthyle ou propyle ;
éventuellement sous la forme des énantiomères individuels, de mélanges des énantiomères individuels ou de racémates, éventuellement sous la forme de leurs sels d'addition acide avec des acides pharmacologiquement inoffensifs et éventuellement sous la forme de solvates et/ou hydrates.

5. Composés de formule 1, selon l'une des revendications 1 à 4, dans laquelle
n est 2 ou 3 ;
m est 1 ou 2 ;
X est CH₂, CO, NR², S ou O ;
A est CO ;
B est un groupe à double liaison choisi dans le groupe comprenant CH₂-O, CH=CH ou CH₂-CH₂ ;
R¹ est H, un groupe méthyle, éthyle, propyle, CF₃, CH₂F ou CH₂CF₃ ;
R² est H, un groupe méthyle, éthyle ou propyle ;
éventuellement sous la forme des énantiomères individuels, de mélanges des énantiomères individuels ou de racémates, éventuellement sous la forme de leurs sels d'addition acide avec des acides pharmacologiquement inoffensifs et éventuellement sous la forme de solvates et/ou hydrates.

6. Composés de formule 1, selon l'une des revendications 1 à 5, dans laquelle
n est 2 ou 3
m est 1 ;
X est CH₂, CO, NR², S ou O ;
A est CO ;
B est un groupe à double liaison choisi dans le groupe comprenant CH₂-O, CH=CH ou CH₂-CH₂ ;
R¹ est H, un groupe méthyle ou CF₃ ;
R² est H ou un groupe méthyle ;
éventuellement sous la forme des énantiomères individuels, de mélanges des énantiomères individuels ou de racémates, éventuellement sous la forme de leurs sels d'addition acide avec des acides pharmacologiquement inoffensifs et éventuellement sous la forme de solvates et/ou hydrates.

7. Composés de formule 1, selon l'une des revendications 1 à 6, dans laquelle
X est NR², O ; où R² a la signification indiquée dans les revendications 1 à 7 ;
éventuellement sous la forme des énantiomères individuels, de mélanges des énantiomères individuels ou de racémates, éventuellement sous la forme de leurs sels d'addition acide avec des acides pharmacologiquement inoffensifs et éventuellement sous la forme de solvates et/ou hydrates.

8. Composés de formule 1, selon l'une des revendications 1 à 7, dans laquelle
n est 2 ou 3 ;
m est 1 ;
X est NR², O ;
A est CO ;
B est un groupe à double liaison choisi dans le groupe comprenant CH₂-O ou CH=CH ;
R¹ est H, un groupe méthyle ou CF₃ ;
R² est H ou un groupe méthyle ;
éventuellement sous la forme des énantiomères individuels, de mélanges des énantiomères individuels ou de racémates, éventuellement sous la forme de leurs sels d'addition acide avec des acides pharmacologiquement inoffensifs et éventuellement sous la forme de solvates et/ou hydrates.

9. Composés de formule 1, selon l'une des revendications 1 à 8, dans laquelle
n est 2 ;
m est 1 ;
X est NH ;
A est CO ;
B est un groupe à double liaison CH₂-O ;
R¹ est H, un groupe méthyle ou CF₃ ;
éventuellement sous la forme des énantiomères individuels, de mélanges des énantiomères individuels ou de racémates, éventuellement sous la forme de leurs sels d'addition acide avec des acides pharmacologiquement inoffensifs et éventuellement sous la forme de solvates et/ou hydrates.

10. Composés de formule 1 selon l'une des revendications 1 à 9, **caractérisés en ce qu'**ils se présentent sous la forme de l'un de leurs sels d'addition acides avec des acides pharmacologiquement inoffensifs qui sont choisis dans le groupe comprenant un chlorhydrate, un bromhydrate, un iodhydrate, un hydrosulfate, un hydrophosphate, un hydrométhanesulfonate, un hydronitrate, un hydromaléate, un hydroacétate, un hydrobenzoate, un hydrocitrate, un hydrofumarate, un hydrotartrate, un hydro-oxalate, un hydrosuccinate, un hydrobenzoate et un hydro-p-toluènesulfonate.

11. Composés de formule 1 selon l'une des revendications 1 à 10, **caractérisés en ce qu'**ils se présentent sous la forme des énantiomères R de formule R-1

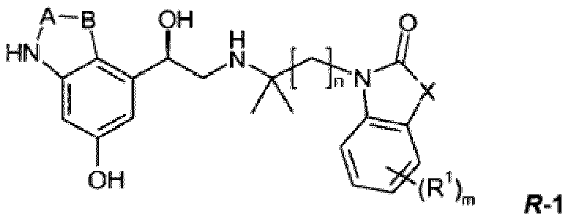

12. Composés de formule 1 selon l'une des revendications 1 à 11 comme médicament.

13. Utilisation des composés de formule 1 selon l'une des revendications 1 à 12, pour la production d'un médicament pour le traitement des maladies des voies respiratoires.

14. Composition pharmaceutique, **caractérisée par** une teneur en composé de formule 1 selon l'une des revendications 1 à 12.
